# EUROPEAN PATENT SPECIFICATION

(11) **EP 3 891 136 B1**
(45) Date of publication and mention of the grant of the patent: **19.07.2023**
(21) Application number: 19809843.6
(22) Date of filing: 29.11.2019
(51) Int. Cl.: C07D 401/06, C07D 401/14, A61K 31/454, A61P 35/00

(54) **4-HETEROARYLCARBONYL-N-(PHENYL OR HETEROARYL)PIPERIDINE-1-CARBOXAMIDES AS INHIBITORS OF TANKYRASES**
4-HETEROARYLCARBONYL-N-(PHENYL- ODER -HETEROARYL)PIPERIDIN-1-CARBOXAMIDE ALS INHIBITOREN VON TANKYRASEN
4-HÉTÉROARYLCARBONYL-N-(PHÉNYLE OU HÉTÉROARYL) PIPÉRIDINE-1-CARBOXAMIDES SERVANT D'INHIBITEURS DE TANKYRASES

(30) Priority: 03.12.2018 EP 18209726
(43) Date of publication of application: 13.10.2021
(73) Proprietor: Merck Patent GmbH, 64293 Darmstadt (DE)
(72) Inventor: BUCHSTALLER, Hans-Peter, 64347 Griesheim (DE); ROHDICH, Felix, 64283 Darmstadt (DE)
(86) International application number: PCT/EP2019/083014
(87) International publication number: WO 2020/114892

(56) References cited:
- WO-A1-2015/018475

## Description

### BACKGROUND OF THE INVENTION

The invention had the object of finding novel compounds having valuable properties, in particular those which can be used for the preparation of medicaments.

Any references in the description to methods of treatment refer to the compounds, pharmaceutical compositions and medicaments of the present invention for use in a method for treatment of the human (or animal) body by therapy.

The present invention relates to carboxylic acid derivatives which inhibit the activity of tankyrases (TANKs). The compounds of this invention are therefore useful in treating diseases such as cancer, multiple sclerosis, cardiovascular diseases, central nervous system injury and different forms of inflammation. The present invention also provides methods for preparing these compounds, pharmaceutical compositions comprising these compounds, and methods of treating diseases utilizing pharmaceutical compositions comprising these compounds.

The enzymes tankyrases consist of TANKs, such as, for example: TANK-1 and TANK-2.

TANK-1 seems to be required for the polymerization of mitotic spindle-associated poly(ADP-ribose). The poly(ADP-ribosyl)ation activity of TANK-1 might be crucial for the accurate formation and maintenance of spindle bipolarity. Inhibition of tankyrases is expected to have a cytotoxic effect on proliferating tumor cells (WO 2008/107478).

Defects in conserved signaling pathways are well known to play key roles in the origins and behavior of essentially all cancers (E.A.Fearon, Cancer Cell, Vol. 16, Issue 5, 2009, 366-368). The Wnt pathway is a target for anti-cancer therapy. A key feature of the Wnt pathway is the regulated proteolysis (degradation) of β-catenin by the β-catenin destruction complex. Proteins like WTX, APC or Axin are involved in the degradation process. A proper degradation of β-catenin is important to avoid an inappropriate activation of the Wnt pathway which has been observed in many cancers. Tankyrases inhibit activity of Axin and hence inhibit the degradation of β-catenin. Consequently, tankyrase inhibitors increase degradation of β-catenin. A paper in the journal *Nature* not only offers important new insights into proteins regulating Wnt signaling but also further supports the approach to antagonize β-catenin levels and localization via small molecules (Huang et al., 2009; Nature, Vol 461, 614-620). The compound XAV939 inhibits growth of DLD-1-cancer cells. They found that XAV9393 blocked Wnt-stimulated accumulation of β-catenin by increasing the levels of the AXIN1 and AXIN2 proteins. Subsequent work by the authors established that XAV939 regulates AXIN levels via inhibition of tankyrases 1 and 2 (TNKS1 and TNKS2), both of which are members of the poly(ADP-ribose) polymerase (PARP) protein family (S.J. Hsiao et al., Biochimie 90, 2008, 83-92).

By inhibition of tankyrase Axin2 is stabilized and increases remyelination after multiple sclerosis lesions (Fancy et al., 2011; Nature, Vol 14, 1009-1016).

It has been found that the compounds according to the invention and salts thereof have very valuable pharmacological properties while being well tolerated.

The present invention specifically relates to compounds of the formula I which inhibit Tankyrase 1 and 2, to compositions which comprise these compounds, and to processes for the use thereof for the treatment of TANK-induced diseases and complaints.

The compounds of the formula I can furthermore be used for the isolation and investigation of the activity or expression of TANKs. In addition, they are particularly suitable for use in diagnostic methods for diseases in connection with unregulated or disturbed TANK activity.

The host or patient can belong to any mammalian species, for example a primate species, particularly humans; rodents, including mice, rats and hamsters; rabbits; horses, cows, dogs, cats, etc. Animal models are of interest for experimental investigations, providing a model for treatment of human disease.

The susceptibility of a particular cell to treatment with the compounds according to the invention can be determined by in vitro tests. Typically, a culture of the cell is combined with a compound according to the invention at various concentrations for a period of time which is sufficient to allow active agents such as anti IgM to induce a cellular response such as expression of a surface marker, usually between about one hour and one week. In vitro testing can be carried out using cultivated cells from blood or from a biopsy sample. The amount of surface marker expressed is assessed by flow cytometry using specific antibodies recognising the marker.

The dose varies depending on the specific compound used, the specific disease, the patient status, etc. A therapeutic dose is typically sufficient considerably to reduce the undesired cell population in the target tissue while the viability of the patient is maintained. The treatment is generally continued until a considerable reduction has occurred, for example an at least about 50% reduction in the cell burden, and may be continued until essentially no more undesired cells are detected in the body.

### PRIOR ART

E. Wahlberg et al., Nature Biotechnology (2012), 30(3), 283.

M. D. Shultz et al., Journal of Medicinal Chemistry 2013 , 56 (16), 6495-6511.

In the same publication, the following benzoylpiperidine derivative is described as Tankyrase inhibitor:

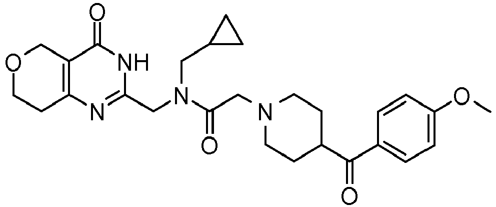

The compounds of the present invention are significantly more active.

Other tankyrase inhibitors are described in WO 2013/012723, WO 2013/010092, WO 2013/008217 and in WO 2015/018475.

Oxoquinazolinyl-butanamide derivatives for the treatment of cancer are described in WO 2015/014442 A1.

### SUMMARY OF THE INVENTION

The invention relates to compounds of the formula I 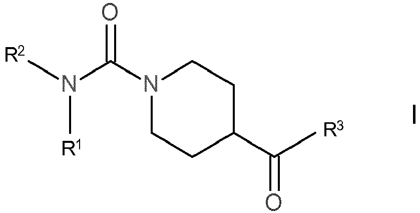 in which
- R¹: denotes A,
- R²: denotes Ar or Het¹,
- R³: denotes Het²,
- Ar: denotes phenyl, which is unsubstituted or mono-, di- or trisubstituted by Hal, CN, A, OR⁴, (CH₂)ₘN(R⁴)₂, SO₂N(R⁴)₂, COOR⁴ and/or CON(R⁴)₂,
- Het¹: denotes pyridyl, pyrimidyl, pyrazinyl or pyridazinyl, each of which is unsubstituted or mono- or disubstituted by Hal, CN, A, NO₂, (CH₂)ₘOR⁴, (CH₂)ₘN(R⁴)₂, S(O)ₘR⁴, SO₂N(R⁴)₂, (CH₂)ₘCOOR⁴ and/or (CH₂)ₘCON(R⁴)₂,
- Het²: denotes benzimidazolyl, benzotriazolyl, indazolyl or indolyl, each of which is unsubstituted or mono-, di- or trisubstituted by Hal, CN, A, NO₂, (CH₂)ₘOR⁴, (CH₂)ₘN(R⁴)₂, S(O)ₘR⁴, SO₂N(R⁴)₂, (CH₂)ₘCOOR⁴ and/or (CH₂)ₘCON(R⁴)₂,
- A: denotes unbranched or branched alkyl with 1, 2, 3, 4, 5, 6, 7 or 8 C-Atoms, wherein one or two non-adjacent CH- and/or CH₂-groups may be replaced by N- or O-atoms and wherein 1-7 H-atoms may be replaced by F, Cl and/or OH,
- R⁴: denotes H or unbranched or branched alkyl with 1, 2, 3 or 4 C-Atoms,
- Hal: denotes F, Cl, Br or I,
- m: denotes 0, 1 or 2,
and pharmaceutically acceptable solvates, salts, tautomers and stereoisomers thereof,
including mixtures thereof in all ratios.

The invention also relates to the optically active forms (stereoisomers), the enantiomers, the racemates, the diastereomers and the hydrates and solvates of these compounds.

Moreover, the invention relates to pharmaceutically acceptable salts of compounds of formula I.

The term solvates of the compounds is taken to mean adductions of inert solvent molecules onto the compounds which form owing to their mutual attractive force. Solvates are, for example, mono- or dihydrates or alkoxides.

It is understood, that the invention also relates to the solvates of the salts.

Also mentioned here but not part of the invention are prodrugs of the compounds of formula I.

As used herein and unless otherwise indicated, the term "prodrug" means a derivative of a compound of formula I that can hydrolyze, oxidize, or otherwise react under biological conditions (in vitro or in vivo) to provide an active compound, particularly a compound of formula I. Examples of prodrugs include, but are not limited to, derivatives and metabolites of a compound of formula I that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. In certain embodiments, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well- known methods.

The expression "effective amount" denotes the amount of a medicament or of a pharmaceutical active ingredient which causes in a tissue, system, animal or human a biological or medical response which is sought or desired, for example, by a researcher or physician.

In addition, the expression "therapeutically effective amount" denotes an amount which, compared with a corresponding subject who has not received this amount, has the following consequence:
improved treatment, healing, prevention or elimination of a disease, syndrome, condition, complaint, disorder or side-effects or also the reduction in the advance of a disease, complaint or disorder.

The expression "therapeutically effective amount" also encompasses the amounts which are effective for increasing normal physiological function.

The invention also relates to the use of mixtures of the compounds of the formula I, for example mixtures of two diastereomers, for example in the ratio 1:1, 1:2, 1:3, 1:4, 1:5, 1:10, 1:100 or 1:1000.

These are particularly preferably mixtures of stereoisomeric compounds.

"Tautomers" refers to isomeric forms of a compound that are in equilibrium with each other. The concentrations of the isomeric forms will depend on the environment the compound is found in and may be different depending upon, for example, whether the compound is a solid or is in an organic or aqueous solution.

The invention relates to the compounds of the formula I and salts thereof and to a process for the preparation of some compounds of formula I and pharmaceutically acceptable salts, solvates, tautomers and stereoisomers thereof, characterised in that a compound of formula I, in which
R³ denotes benzimidazolyl, benzotriazolyl, indazolyl or indolyl, each of which is substituted by (CH₂)ₘCOOR⁴,
R⁴ denotes unbranched or branched alkyl with 1, 2, 3 or 4 C-Atoms,
and R¹ and R² have meanings indicated in claim 1,
is converted into another compound of formula I, in which
R³ denotes benzimidazolyl, benzotriazolyl, indazolyl or indolyl, each of which is substituted by (CH₂)ₘCOOR⁴,
R⁴ denotes H,
and R¹ and R² have meanings indicated in claim 1,
by hydrolyzing the ester;
   and/or
a base or acid of the formula I is converted into one of its salts.

Above and below, the radicals R¹, R², R³ have the meanings indicated for the formula I, unless explicitely stated otherwise.

A denotes alkyl, this is unbranched (linear) or branched, and has 1, 2, 3, 4, 5, 6, 7 or 8 C atoms. A preferably denotes methyl, ethyl, propyl, isopropyl, butyl, isobutyl, sec-butyl or tert-butyl, furthermore also pentyl, 1-, 2- or 3-methylbutyl, 1,1- , 1,2- or 2,2-dimethylpropyl, 1-ethylpropyl, hexyl, 1- , 2-, 3- or 4-methylpentyl, 1,1- , 1,2- , 1,3- , 2,2- , 2,3- or 3,3-dimethylbutyl, 1- or 2-ethylbutyl, 1-ethyl-1-methylpropyl, 1-ethyl-2-methylpropyl, 1,1,2- or 1,2,2-trimethylpropyl, furthermore preferably, for example, trifluoromethyl. A very particularly preferably denotes alkyl having 2, 3, 4, 5 or 6 C atoms, preferably ethyl, propyl, isopropyl, butyl, isobutyl, sec-butyl, tert-butyl, pentyl, hexyl, trifluoromethyl, pentafluoroethyl or 1,1,1-trifluoroethyl. Moreover, A denotes preferably CH₂OCH₃, CH₂CH₂OH or CH₂CH₂OCH₃.

Het¹ preferably denotes pyridyl, which is unsubstituted or mono- or disubstituted by (CH₂)ₘCOOR⁴.

Ar preferably denotes phenyl, which is unsubstituted or mono-, di- or trisubstituted by Hal, OR⁴ and/or COOR⁴.

Het² preferably denotes benzimidazolyl, benzotriazolyl, indazolyl or indolyl, each of which is unsubstituted or mono-, di- or trisubstituted by A and/or (CH₂)ₘCOOR⁴.

A preferably denotes unbranched or branched alkyl with 1, 2, 3, 4, 5, 6, 7 or 8 C-Atoms, wherein 1-5 H-atoms may be replaced by F.

R⁴ preferably denotes H or methyl.

Throughout the invention, all radicals which occur more than once may be identical or different, i.e. are independent of one another.

The compounds of the formula I may have one or more chiral centres and can therefore occur in various stereoisomeric forms. The formula I encompasses all these forms.

Accordingly, the invention relates, in particular, to the compounds of the formula I in which at least one of the said radicals has one of the preferred meanings indicated above. Some preferred groups of compounds may be expressed by the following sub-formulae Ia to Ie, which conform to the formula I and in which the radicals not designated in greater detail have the meaning indicated for the formula I, but in which

| | | |
|---|---|---|
| in Ia | Het¹ | denotes pyridyl, which is unsubstituted or mono- or disubstituted by (CH₂)ₘCOOR⁴; |
| in Ib | Ar | denotes phenyl, which is unsubstituted or mono-, di- or trisubstituted by Hal, OR⁴ and/or COOR⁴; |
| in Ic | Het² | denotes benzimidazolyl, benzotriazolyl, indazolyl or indolyl, each of which is unsubstituted or mono-, di- or trisubstituted by A and/or (CH₂)ₘCOOR⁴; |
| in Id | A | denotes unbranched or branched alkyl with 1, 2, 3, 4, 5, 6, 7 or 8 C-Atoms, wherein 1-5 H-atoms may be replaced by F; |
| in Ie | R¹ | denotes A, |
| | R² | denotes Ar or Het¹, |
| | R³ | denotes Het², |
| | Ar | denotes phenyl, which is unsubstituted or mono-, di- or trisubstituted by Hal, OR⁴ and/or COOR⁴, |
| | Het¹ | denotes pyridyl, which is unsubstituted or mono- or disubstituted by (CH₂), |
| | Het² | denotes benzimidazolyl, benzotriazolyl, indazolyl or indolyl, each of which is unsubstituted or mono-, di- or trisubstituted by A and/or (CH₂)ₘCOOR⁴, |
| | A | denotes unbranched or branched alkyl with 1, 2, 3, 4, 5, 6, 7 or 8 C-Atoms, wherein 1-5 H-atoms may be replaced by F, |
| | R⁴ | denotes H or unbranched or branched alkyl with 1, 2, 3 or 4 C-Atoms, |
| | Hal | denotes F, Cl, Br or I, |
| | m | denotes 0, 1 or 2, |

and pharmaceutically acceptable salts, tautomers and stereoisomers thereof, including mixtures thereof in all ratios.

The compounds of the formula I and also the starting materials for their preparation are, in addition, prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and suitable for the said reactions. Use can also be made here of variants known per se which are not mentioned here in greater detail.

Moreover, some compounds of the formula I can preferably be obtained by converting a compound of formula I, in which
R³ denotes benzimidazolyl, benzotriazolyl, indazolyl or indolyl, each of which is substituted by (CH₂)ₘCOOR⁴,
R⁴ denotes unbranched or branched alkyl with 1, 2, 3 or 4 C-Atoms, and R¹ and R² have meanings indicated in claim 1,
   into another compound of formula I, in which
   - R³: denotes benzimidazolyl, benzotriazolyl, indazolyl or indolyl, each of which is substituted by (CH₂)ₘCOOR⁴,
   - R⁴: denotes H,
and R¹ and R² have meanings indicated in claim 1,
   by hydrolyzing the ester.

The reaction is generally carried out in water and/or in a suitable inert solvent together with an alkali or alkaline earth metal hydroxide, carbonate or bicarbonate or another salt of a weak acid of the alkali or alkaline earth metals, preferably of potassium, sodium, calcium or caesium.

Particular preference is given to NaOH.

Depending on the conditions used, the reaction time is between a few minutes and 14 days, the reaction temperature is between about -30° and 140°, normally between -10° and 90°, in particular between about 0° and about 70°.

Examples of suitable inert solvents are hydrocarbons, such as hexane, petroleum ether, benzene, toluene or xylene; chlorinated hydrocarbons, such as trichloroethylene, 1,2-dichloroethane, carbon tetrachloride, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or 1,4-dioxane; glycol ethers, such as ethylene glycol monomethyl or monoethyl ether, ethylene glycol dimethyl ether (diglyme); ketones, such as acetone or butanone; amides, such as acetamide, dimethylacetamide or dimethylformamide (DMF); nitriles, such as acetonitrile; sulfoxides, such as dimethyl sulfoxide (DMSO); carbon disulfide; carboxylic acids, such as formic acid or acetic acid; nitro compounds, such as nitromethane or nitrobenzene; esters, such as ethyl acetate, or mixtures of the said solvents.

Particular preference is given to 1,4-dioxane.

### Pharmaceutical salts and other forms

The said compounds according to the invention can be used in their final non-salt form. On the other hand, the present invention also encompasses the use of these compounds in the form of their pharmaceutically acceptable salts, which can be derived from various organic and inorganic acids and bases by procedures known in the art. Pharmaceutically acceptable salt forms of the compounds of the formula I are for the most part prepared by conventional methods. If the compound of the formula I contains a carboxyl group, one of its suitable salts can be formed by reacting the compound with a suitable base to give the corresponding base-addition salt. Such bases are, for example, alkali metal hydroxides, including potassium hydroxide, sodium hydroxide and lithium hydroxide; alkaline earth metal hydroxides, such as barium hydroxide and calcium hydroxide; alkali metal alkoxides, for example potassium ethoxide and sodium propoxide; and various organic bases, such as piperidine, diethanolamine and N-methyl-glutamine. The aluminium salts of the compounds of the formula I are likewise included. In the case of certain compounds of the formula I, acid-addition salts can be formed by treating these compounds with pharmaceutically acceptable organic and inorganic acids, for example hydrogen halides, such as hydrogen chloride, hydrogen bromide or hydrogen iodide, other mineral acids and corresponding salts thereof, such as sulfate, nitrate or phosphate and the like, and alkyl- and monoarylsulfonates, such as ethanesulfonate, toluenesulfonate and benzenesulfonate, and other organic acids and corresponding salts thereof, such as acetate, trifluoroacetate, tartrate, maleate, succinate, citrate, benzoate, salicylate, ascorbate and the like. Accordingly, pharmaceutically acceptable acid-addition salts of the compounds of the formula I include the following: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, di-gluconate, dihydrogenphosphate, dinitrobenzoate, dodecylsulfate, ethanesulfonate, fumarate, formate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, palmoate, pectinate, persulfate, phenylacetate, 3-phenylpropionate, phosphate, phosphonate, phthalate, but this does not represent a restriction.

Furthermore, the base salts of the compounds according to the invention include aluminium, ammonium, calcium, copper, iron(III), iron(II), lithium, magnesium, manganese(III), manganese(II), potassium, sodium and zinc salts, but this is not intended to represent a restriction. Of the above-mentioned salts, preference is given to ammonium; the alkali metal salts sodium and potassium, and the alkaline earth metal salts calcium and magnesium. Salts of the compounds of the formula I which are derived from pharmaceutically acceptable organic non-toxic bases include salts of primary, secondary and tertiary amines, substituted amines, also including naturally occurring substituted amines, cyclic amines, and basic ion exchanger resins, for example arginine, betaine, caffeine, chloroprocaine, choline, N,N'-dibenzylethylenediamine (benzathine), dicyclohexylamine, diethanolamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine and tris-(hydroxymethyl)methylamine (tromethamine), but this is not intended to represent a restriction.

Compounds of the present invention which contain basic nitrogen-containing groups can be quaternised using agents such as (C₁-C₄)alkyl halides, for example methyl, ethyl, isopropyl and tert-butyl chloride, bromide and iodide; di(C₁-C₄)alkyl sulfates, for example dimethyl, diethyl and diamyl sulfate; (C₁₀-C₁₈)alkyl halides, for example decyl, dodecyl, lauryl, myristyl and stearyl chloride, bromide and iodide; and aryl(C₁-C₄)alkyl halides, for example benzyl chloride and phenethyl bromide. Both water- and oil-soluble compounds according to the invention can be prepared using such salts.

The above-mentioned pharmaceutical salts which are preferred include acetate, trifluoroacetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate and tromethamine, but this is not intended to represent a restriction.

Particular preference is given to hydrochloride, dihydrochloride, hydrobromide, maleate, mesylate, phosphate, sulfate and succinate.

The acid-addition salts of basic compounds of the formula I are prepared by bringing the free base form into contact with a sufficient amount of the desired acid, causing the formation of the salt in a conventional manner. The free base can be regenerated by bringing the salt form into contact with a base and isolating the free base in a conventional manner. The free base forms differ in a certain respect from the corresponding salt forms thereof with respect to certain physical properties, such as solubility in polar solvents; for the purposes of the invention, however, the salts otherwise correspond to the respective free base forms thereof.

As mentioned, the pharmaceutically acceptable base-addition salts of the compounds of the formula I are formed with metals or amines, such as alkali metals and alkaline earth metals or organic amines. Preferred metals are sodium, potassium, magnesium and calcium. Preferred organic amines are N,N'-dibenzylethylenediamine, chloroprocaine, choline, diethanolamine, ethylenediamine, N-methyl-D-glucamine and procaine.

The base-addition salts of acidic compounds according to the invention are prepared by bringing the free acid form into contact with a sufficient amount of the desired base, causing the formation of the salt in a conventional manner. The free acid can be regenerated by bringing the salt form into contact with an acid and isolating the free acid in a conventional manner. The free acid forms differ in a certain respect from the corresponding salt forms thereof with respect to certain physical properties, such as solubility in polar solvents; for the purposes of the invention, however, the salts otherwise correspond to the respective free acid forms thereof.

If a compound according to the invention contains more than one group which is capable of forming pharmaceutically acceptable salts of this type, the invention also encompasses multiple salts. Typical multiple salt forms include, for example, bitartrate, diacetate, difumarate, dimeglumine, diphosphate, disodium and trihydrochloride, but this is not intended to represent a restriction.

With regard to that stated above, it can be seen that the expression "pharmaceutically acceptable salt" in the present connection is taken to mean an active ingredient which comprises a compound of the formula I in the form of one of its salts, in particular if this salt form imparts improved pharmacokinetic properties on the active ingredient compared with the free form of the active ingredient or any other salt form of the active ingredient used earlier. The pharmaceutically acceptable salt form of the active ingredient can also provide this active ingredient for the first time with a desired pharmacokinetic property which it did not have earlier and can even have a positive influence on the pharmacodynamics of this active ingredient with respect to its therapeutic efficacy in the body.

### Isotopes

There is furthermore intended that a compound of the formula I includes isotope-labelled forms thereof. An isotope-labelled form of a compound of the formula I is identical to this compound apart from the fact that one or more atoms of the compound have been replaced by an atom or atoms having an atomic mass or mass number which differs from the atomic mass or mass number of the atom which usually occurs naturally. Examples of isotopes which are readily commercially available and which can be incorporated into a compound of the formula I by well-known methods include isotopes of hydrogen, carbon, nitrogen, oxygen, phosphorus, fluorine and chlorine, for example ²H, ³H, ¹³C, ¹⁴C, ¹⁵N, ¹⁸O, ¹⁷O, ³¹P, ³²P, ³⁵S, ¹⁸F and ³⁶Cl, respectively. A compound of the formula I, or a pharmaceutically acceptable salt thereof which contains one or more of the above-mentioned isotopes and/or other iso-topes of other atoms is intended to be part of the present invention. An isotope-labelled compound of the formula I can be used in a number of beneficial ways. For example, an isotope-labelled compound of the formula I into which, for example, a radioisotope, such as ³H or ¹⁴C, has been incorporated is suitable for medicament and/or substrate tissue distribution assays. These radioisotopes, i.e. tritium (³H) and carbon-14 (¹⁴C), are particularly preferred owing to simple preparation and excellent detectability. Incorporation of heavier isotopes, for example deuterium (²H), into a compound of the formula I has therapeutic advantages owing to the higher metabolic stability of this isotope-labelled compound. Higher metabolic stability translates directly into an increased in vivo half-life or lower dosages, which under most circumstances would represent a preferred embodiment of the present invention. An isotope-labelled compound of the formula I can usually be prepared by carrying out the procedures disclosed in the synthesis schemes and the related description, in the example part and in the preparation part in the present text, replacing a non-isotope-labelled reactant by a readily available isotope-labelled reactant.

Deuterium (²H) can also be incorporated into a compound of the formula I for the purpose in order to manipulate the oxidative metabolism of the compound by way of the primary kinetic isotope effect. The primary kinetic isotope effect is a change of the rate for a chemical reaction that results from exchange of isotopic nuclei, which in turn is caused by the change in ground state energies necessary for covalent bond formation after this isotopic exchange. Exchange of a heavier isotope usually results in a lowering of the ground state energy for a chemical bond and thus cause a reduction in the rate in rate-limiting bond breakage. If the bond breakage occurs in or in the vicinity of a saddle-point region along the coordinate of a multi-product reaction, the product distribution ratios can be altered substantially. For explanation: if deuterium is bonded to a carbon atom at a non-exchangeable position, rate differences of k_{M}/k_{D} = 2-7 are typical. If this rate difference is successfully applied to a compound of the formula I that is susceptible to oxidation, the profile of this compound in vivo can be drastically modified and result in improved pharmacokinetic properties.

When discovering and developing therapeutic agents, the person skilled in the art attempts to optimise pharmacokinetic parameters while retaining desirable in vitro properties. It is reasonable to assume that many compounds with poor pharmacokinetic profiles are susceptible to oxidative metabolism. In vitro liver microsomal assays currently available provide valuable information on the course of oxidative metabolism of this type, which in turn permits the rational design of deuterated compounds of the formula I with improved stability through resistance to such oxidative meta-bolism. Significant improvements in the pharmacokinetic profiles of compounds of the formula I are thereby obtained, and can be expressed quantitatively in terms of increases in the in vivo half-life (t1/2), concen-tra-tion at maximum therapeutic effect (Cₘₐₓ), area under the dose response curve (AUC), and F; and in terms of reduced clearance, dose and materi-als costs.

The following is intended to illustrate the above: a compound of the formula I which has multiple potential sites of attack for oxidative metabolism, for example benzylic hydrogen atoms and hydrogen atoms bonded to a nitrogen atom, is prepared as a series of analogues in which various combinations of hydrogen atoms are replaced by deuterium atoms, so that some, most or all of these hydrogen atoms have been replaced by deuterium atoms. Half-life determinations enable favourable and accurate determination of the extent of the extent to which the improve-ment in resistance to oxidative metabolism has improved. In this way, it is deter-mined that the half-life of the parent compound can be extended by up to 100% as the result of deuterium-hydrogen exchange of this type.

Deuterium-hydrogen exchange in a compound of the formula I can also be used to achieve a favourable modification of the metabolite spectrum of the starting compound in order to diminish or eliminate undesired toxic metabolites. For example, if a toxic metabolite arises through oxidative carbon-hydrogen (C-H) bond cleavage, it can reasonably be assumed that the deuterated analogue will greatly diminish or eliminate production of the unwanted metabolite, even if the particular oxidation is not a rate-determining step. Further information on the state of the art with respect to deuterium-hydrogen exchange may be found, for example in Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al, Biochemistry 33(10) 2927-2937, 1994, and Jarman et al. Carcinogenesis 16(4), 683-688, 1993.

The invention furthermore relates to medicaments comprising at least one compound of the formula I and/or pharmaceutically acceptable derivatives, solvates and stereoisomers thereof, including mixtures thereof in all ratios, and optionally excipients and/or adjuvants.

Pharmaceutical formulations can be administered in the form of dosage units which comprise a predetermined amount of active ingredient per dosage unit. Such a unit can comprise, for example, 0.5 mg to 1 g, preferably 1 mg to 700 mg, particularly preferably 5 mg to 100 mg, of a compound according to the invention, depending on the condition treated, the method of administration and the age, weight and condition of the patient, or pharmaceutical formulations can be administered in the form of dosage units which comprise a predetermined amount of active ingredient per dosage unit. Preferred dosage unit formulations are those which comprise a daily dose or part-dose, as indicated above, or a corresponding fraction thereof of an active ingredient. Furthermore, pharmaceutical formulations of this type can be prepared using a process which is generally known in the pharmaceutical art.

Pharmaceutical formulations can be adapted for administration via any desired suitable method, for example by oral (including buccal or sublingual), rectal, nasal, topical (including buccal, sublingual or transdermal), vaginal or parenteral (including subcutaneous, intramuscular, intravenous or intradermal) methods. Such formulations can be prepared using all processes known in the pharmaceutical art by, for example, combining the active ingredient with the excipient(s) or adjuvant(s).

Pharmaceutical formulations adapted for oral administration can be administered as separate units, such as, for example, capsules or tablets; powders or granules; solutions or suspensions in aqueous or non-aqueous liquids; edible foams or foam foods; or oil-in-water liquid emulsions or water-in-oil liquid emulsions.

Thus, for example, in the case of oral administration in the form of a tablet or capsule, the active-ingredient component can be combined with an oral, non-toxic and pharmaceutically acceptable inert excipient, such as, for example, ethanol, glycerol, water and the like. Powders are prepared by comminuting the compound to a suitable fine size and mixing it with a pharmaceutical excipient comminuted in a similar manner, such as, for example, an edible carbohydrate, such as, for example, starch or mannitol. A flavour, preservative, dispersant and dye may likewise be present.

Capsules are produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the compound comminuted in a suitable manner with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbant, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tabletting machine, giving lumps of non-uniform shape, which are broken up to form granules. The granules can be lubricated by addition of stearic acid, a stearate salt, talc or mineral oil in order to prevent sticking to the tablet casting moulds. The lubricated mixture is then pressed to give tablets. The compounds according to the invention can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

Oral liquids, such as, for example, solution, syrups and elixirs, can be prepared in the form of dosage units so that a given quantity comprises a prespecified amount of the compound. Syrups can be prepared by dissolving the compound in an aqueous solution with a suitable flavour, while elixirs are prepared using a non-toxic alcoholic vehicle. Suspensions can be formulated by dispersion of the compound in a non-toxic vehicle. Solubilisers and emulsifiers, such as, for example, ethoxylated isostearyl alcohols and polyoxyethylene sorbitol ethers, preservatives, flavour additives, such as, for example, peppermint oil or natural sweeteners or saccharin, or other artificial sweeteners and the like, can likewise be added.

The dosage unit formulations for oral administration can, if desired, be encapsulated in microcapsules. The formulation can also be prepared in such a way that the release is extended or retarded, such as, for example, by coating or embedding of particulate material in polymers, wax and the like.

The compounds of the formula I and pharmaceutically salts, tautomers and stereoisomers thereof can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

The compounds of the formula I and the salts, tautomers and stereoisomers thereof can also be delivered using monoclonal antibodies as individual carriers to which the compound molecules are coupled. The compounds can also be coupled to soluble polymers as targeted medicament carriers. Such polymers may encompass polyvinylpyrrolidone, pyran copolymer, polyhydroxypropylmethacrylamidophenol, polyhydroxyethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, polyorthoesters, polyacetals, polydihydroxypyrans, polycyanoacrylates and crosslinked or amphipathic block copolymers of hydrogels.

Pharmaceutical formulations adapted for transdermal administration can be administered as independent plasters for extended, close contact with the epidermis of the recipient. Thus, for example, the active ingredient can be delivered from the plaster by iontophoresis.

Pharmaceutical compounds adapted for topical administration can be formulated as ointments, creams, suspensions, lotions, powders, solutions, pastes, gels, sprays, aerosols or oils.

For the treatment of the eye or other external tissue, for example mouth and skin, the formulations are preferably applied as topical ointment or cream. In the case of formulation to give an ointment, the active ingredient can be employed either with a paraffinic or a water-miscible cream base. Alternatively, the active ingredient can be formulated to give a cream with an oil-in-water cream base or a water-in-oil base.

Pharmaceutical formulations adapted for topical application to the eye include eye drops, in which the active ingredient is dissolved or suspended in a suitable carrier, in particular an aqueous solvent.

Pharmaceutical formulations adapted for topical application in the mouth encompass lozenges, pastilles and mouthwashes.

Pharmaceutical formulations adapted for rectal administration can be administered in the form of suppositories or enemas.

Pharmaceutical formulations adapted for nasal administration in which the carrier substance is a solid comprise a coarse powder having a particle size, for example, in the range 20-500 microns, which is administered in the manner in which snuff is taken, i.e. by rapid inhalation via the nasal passages from a container containing the powder held close to the nose. Suitable formulations for administration as nasal spray or nose drops with a liquid as carrier substance encompass active-ingredient solutions in water or oil.

Pharmaceutical formulations adapted for administration by inhalation encompass finely particulate dusts or mists, which can be generated by various types of pressurised dispensers with aerosols, nebulisers or insufflators.

Pharmaceutical formulations adapted for vaginal administration can be administered as pessaries, tampons, creams, gels, pastes, foams or spray formulations.

Pharmaceutical formulations adapted for parenteral administration include aqueous and non-aqueous sterile injection solutions comprising antioxidants, buffers, bacteriostatics and solutes, by means of which the formulation is rendered isotonic with the blood of the recipient to be treated; and aqueous and non-aqueous sterile suspensions, which may comprise suspension media and thickeners. The formulations can be administered in single-dose or multidose containers, for example sealed ampoules and vials, and stored in freeze-dried (lyophilised) state, so that only the addition of the sterile carrier liquid, for example water for injection purposes, immediately before use is necessary. Injection solutions and suspensions prepared in accordance with the recipe can be prepared from sterile powders, granules and tablets.

It goes without saying that, in addition to the above particularly mentioned constituents, the formulations may also comprise other agents usual in the art with respect to the particular type of formulation; thus, for example, formulations which are suitable for oral administration may comprise flavours.

A therapeutically effective amount of a compound of the formula I depends on a number of factors, including, for example, the age and weight of the animal, the precise condition that requires treatment, and its severity, the nature of the formulation and the method of administration, and is ultimately determined by the treating doctor or vet. However, an effective amount of a compound according to the invention is generally in the range from 0.1 to 100 mg/kg of body weight of the recipient (mammal) per day and particularly typically in the range from 1 to 10 mg/kg of body weight per day. Thus, the actual amount per day for an adult mammal weighing 70 kg is usually between 70 and 700 mg, where this amount can be administered as a single dose per day or usually in a series of part-doses (such as, for example, two, three, four, five or six) per day, so that the total daily dose is the same. An effective amount of a salt or solvate or of a physiologically functional derivative thereof can be determined as the fraction of the effective amount of the compound according to the invention *per se.* It can be assumed that similar doses are suitable for the treatment of other conditions mentioned above.

A combined treatment of this type can be achieved with the aid of simultaneous, consecutive or separate dispensing of the individual components of the treatment. Combination products of this type employ the compounds according to the invention.

The invention furthermore relates to medicaments comprising at least one compound of the formula I and/or pharmaceutically acceptable salts, tautomers and stereoisomers thereof, including mixtures thereof in all ratios, and at least one further medicament active ingredient.

The invention also relates to a set (kit) consisting of separate packs of
(a) an effective amount of a compound of the formula I and/or pharmaceutically acceptable salts, tautomers and stereoisomers thereof, including mixtures thereof in all ratios,
   and
(b) an effective amount of a further medicament active ingredient.

The set comprises suitable containers, such as boxes, individual bottles, bags or ampoules. The set may, for example, comprise separate ampoules, each containing an effective amount of a compound of the formula I and/or pharmaceutically acceptable salts, tautomers and stereoisomers thereof, including mixtures thereof in all ratios,
and an effective amount of a further medicament active ingredient in dissolved or lyophilised form.

"Treating" as used herein, means an alleviation, in whole or in part, of symptoms associated with a disorder or disease, or slowing, or halting of further progression or worsening of those symptoms, or prevention or prophylaxis of the disease or disorder in a subject at risk for developing the disease or disorder.

The term "effective amount" in connection with a compound of formula (I) can mean an amount capable of alleviating, in whole or in part, symptoms associated with a disorder or disease, or slowing or halting further progression or worsening of those symptoms, or preventing or providing prophylaxis for the disease or disorder in a subject having or at risk for developing a disease disclosed herein, such as inflammatory conditions, immunological conditions, cancer or metabolic conditions.

In one embodiment an effective amount of a compound of formula (I) is an amount that inhibits a tankyrase in a cell, such as, for example, in vitro or in vivo. In some embodiments, the effective amount of the compound of formula (I) inhibits tankyrase in a cell by 10%, 20%, 30%, 40%, 50%, 60%, 70%, 80%, 90% or 99%, compared to the activity of tankyrase in an untreated cell. The effective amount of the compound of formula (I), for example in a pharmaceutical composition, may be at a level that will exercise the desired effect; for example, about 0.005 mg/kg of a subject's body weight to about 10 mg/kg of a subject's body weight in unit dosage for both oral and parenteral administration.

### USE

The present compounds are suitable as pharmaceutical active ingredients for mammals, especially for humans, in the treatment of cancer, multiple sclerosis, cardiovascular diseases, central nervous system injury and different forms of inflammation.

The present invention encompasses the use of the compounds of the formula I and/or pharmaceutically acceptable salts, tautomers and stereoisomers thereof for the preparation of a medicament for the treatment or prevention of cancer, multiple sclerosis, cardiovascular diseases, central nervous system injury and different forms of inflammation.

Examples of inflammatory diseases include rheumatoid arthritis, psoriasis, contact dermatitis, delayed hypersensitivity reaction and the like.

Also encompassed is the use of the compounds of the formula I and/or pharmaceutically acceptable salts, tautomers and stereoisomers thereof for the preparation of a medicament for the treatment or prevention of a tankyrase-induced disease or a tankyrase-induced condition in a mammal, in which to this method a therapeutically effective amount of a compound according to the invention is administered to a sick mammal in need of such treatment. The therapeutic amount varies according to the specific disease and can be determined by the person skilled in the art without undue effort.

The expression "tankyrase-induced diseases or conditions" refers to pathological conditions that depend on the activity of one or more tankyrases. Diseases associated with tankyrase activity include cancer, multiple sclerosis, cardiovascular diseases, central nervous system injury and different forms of inflammation.

The present invention specifically relates to compounds of the formula I and pharmaceutically acceptable salts, tautomers and stereoisomers thereof, including mixtures thereof in all ratios,
for the use for the treatment of diseases in which the inhibition, regulation and/or modulation inhibition of tankyrase plays a role.

The present invention specifically relates to compounds of the formula I and pharmaceutically acceptable salts, tautomers and stereoisomers thereof, including mixtures thereof in all ratios, for the use for the inhibition of tankyrase.

The present invention specifically relates to compounds of the formula I and pharmaceutically acceptable salts, tautomers and stereoisomers thereof, including mixtures thereof in all ratios, for the use for the treatment of cancer, multiple sclerosis, cardiovascular diseases, central nervous system injury and different forms of inflammation.

The present invention specifically relates to methods for treating or preventing cancer, multiple sclerosis, cardiovascular diseases, central nervous system injury and different forms of inflammation, comprising administering to a subject in need thereof an effective amount of a compound of formula I or a pharmaceutically acceptable salt, tautomer, stereoisomer or solvate thereof.

Representative cancers that compounds of formula I are useful for treating or preventing include, but are not limited to, cancer of the head, neck, eye, mouth, throat, esophagus, bronchus, larynx, pharynx, chest, bone, lung, colon, rectum, stomach, prostate, urinary bladder, uterine, cervix, breast, ovaries, testicles or other reproductive organs, skin, thyroid, blood, lymph nodes, kidney, liver, pancreas, brain, central nervous system, solid tumors and blood-borne tumors.

Representative cardiovascular diseases that compounds of formula I are useful for treating or preventing include, but are not limited to, restenosis, atherosclerosis and its consequences such as stroke, myocardial infarction, ischemic damage to the heart, lung, gut, kidney, liver, pancreas, spleen or brain.

The present invention relates to a method of treating a proliferative, autoimmune, anti inflammatory or infectious disease disorder that comprises administering to a subject in need thereof a therapeutically effective amount of a compound of formula I.

Preferably, the present invention relates to a method wherein the disease is a cancer.

Particularly preferable, the present invention relates to a method wherein the disease is a cancer, wherein administration is simultaneous,
sequential or in alternation with administration of at least one other active drug agent.

The disclosed compounds of the formula I can be administered in combination with other known therapeutic agents, including anticancer agents. As used here, the term "anticancer agent" relates to any agent which is administered to a patient with cancer for the purposes of treating the cancer.

The anti-cancer treatment defined above may be applied as a monotherapy or may involve, in addition to the herein disclosed compounds of formula I, conventional surgery or radiotherapy or medicinal therapy. Such medicinal therapy, e.g. a chemotherapy or a targeted therapy, may include one or more, but preferably one, of the following anti-tumor agents:
Alkylating agents
   such as altretamine, bendamustine, busulfan, carmustine, chlorambucil, chlormethine, cyclophosphamide, dacarbazine, ifosfamide, improsulfan, tosilate, lomustine, melphalan, mitobronitol, mitolactol, nimustine, ranimustine, temozolomide, thiotepa, treosulfan, mechloretamine, carboquone; apaziquone, fotemustine, glufosfamide, palifosfamide, pipobroman, trofosfamide, uramustine, TH-302⁴, VAL-083⁴;
Platinum Compounds
   such as carboplatin, cisplatin, eptaplatin, miriplatine hydrate, oxaliplatin, lobaplatin, nedaplatin, picoplatin, satraplatin;
   lobaplatin, nedaplatin, picoplatin, satraplatin;
DNA altering agents
   such as amrubicin, bisantrene, decitabine, mitoxantrone, procarbazine, trabectedin, clofarabine;
   amsacrine, brostallicin, pixantrone, laromustine^{1,3};
Topoisomerase Inhibitors
   such as etoposide, irinotecan, razoxane, sobuzoxane, teniposide, topotecan; amonafide, belotecan, elliptinium acetate, voreloxin;
Microtubule modifiers
   such as cabazitaxel, docetaxel, eribulin, ixabepilone, paclitaxel, vinblastine, vincristine, vinorelbine, vindesine, vinflunine;
   fosbretabulin, tesetaxel;
Antimetabolites
   such as asparaginase³, azacitidine, calcium levofolinate, capecitabine, cladribine, cytarabine, enocitabine, floxuridine, fludarabine, fluorouracil, gemcitabine, mercaptopurine, methotrexate, nelarabine, pemetrexed, pralatrexate, azathioprine, thioguanine, carmofur;
   doxifluridine, elacytarabine, raltitrexed, sapacitabine, tegafur^{2,3}, trimetrexate;
Anticancer antibiotics
   such as bleomycin, dactinomycin, doxorubicin, epirubicin, idarubicin, levamisole, miltefosine, mitomycin C, romidepsin, streptozocin, valrubicin, zinostatin, zorubicin, daunurobicin, plicamycin;
   aclarubicin, peplomycin, pirarubicin;
Hormones/Antagonists
   such as abarelix, abiraterone, bicalutamide, buserelin, calusterone, chlorotrianisene, degarelix, dexamethasone, estradiol, fluocortolone fluoxymesterone, flutamide, fulvestrant, goserelin, histrelin, leuprorelin, megestrol, mitotane, nafarelin, nandrolone, nilutamide, octreotide, prednisolone, raloxifene, tamoxifen, thyrotropin alfa, toremifene, trilostane, triptorelin, diethylstilbestrol;
   acolbifene, danazol, deslorelin, epitiostanol, orteronel, enzalutamide^{1,3};
Aromatase inhibitors
   such as aminoglutethimide, anastrozole, exemestane, fadrozole, letrozole, testolactone;
   formestane;
Small molecule kinase inhibitors
   such as crizotinib, dasatinib, erlotinib, imatinib, lapatinib, nilotinib, pazopanib, regorafenib, ruxolitinib, sorafenib, sunitinib, vandetanib, vemurafenib, bosutinib, gefitinib, axitinib;
   afatinib, alisertib, dabrafenib, dacomitinib, dinaciclib, dovitinib, enzastaurin, nintedanib, lenvatinib, linifanib, linsitinib, masitinib, midostaurin, motesanib, neratinib, orantinib, perifosine, ponatinib, radotinib, rigosertib, tipifarnib, tivantinib, tivozanib, trametinib, pimasertib, brivanib alaninate, cediranib, apatinib⁴, cabozantinib S-malate^{1,3}, ibrutinib^{1,3}, icotinib⁴, buparlisib², cipatinib⁴, cobimetinib^{1,3}, idelalisib^{1,3}, fedratinib', XL-647⁴;
Photosensitizers
   such as methoxsalen³;
   porfimer sodium, talaporfin, temoporfin;
Antibodies
   such as avelumab, alemtuzumab, besilesomab, brentuximab vedotin, cetuximab, denosumab, ipilimumab, ofatumumab, panitumumab, rituximab, tositumomab, trastuzumab, bevacizumab, pertuzumab^{2,3};
   catumaxomab, elotuzumab, epratuzumab, farletuzumab, mogamulizumab, necitumumab, nimotuzumab, obinutuzumab, ocaratuzumab, oregovomab, ramucirumab, rilotumumab, siltuximab, tocilizumab, zalutumumab, zanolimumab, matuzumab, dalotuzumab^{1,2,3}, onartuzumab^{1,3}, racotumomab¹, tabalumab^{1,3}, EMD-525797⁴, nivolumab^{1,3};
Cytokines
   such as aldesleukin, interferon alfa², interferon alfa2a³, interferon alfa2b^{2,3}; celmoleukin, tasonermin, teceleukin, oprelvekin^{1,3}, recombinant interferon beta-1a⁴;
Drug Conjugates
   such as denileukin diftitox, ibritumomab tiuxetan, iobenguane I123, prednimustine, trastuzumab emtansine, estramustine, gemtuzumab, ozogamicin, aflibercept;
   cintredekin besudotox, edotreotide, inotuzumab ozogamicin, naptumomab estafenatox, oportuzumab monatox, technetium (99mTc) arcitumomab^{1,3}, vintafolide^{1,3};
Vaccines
   such as sipuleucel³; vitespen³, emepepimut-S³, oncoVAX⁴, rindopepimut³, troVax⁴, MGN-1601⁴, MGN-1703⁴;
Miscellaneous
   alitretinoin, bexarotene, bortezomib, everolimus, ibandronic acid, imiquimod, lenalidomide, lentinan, metirosine, mifamurtide, pamidronic acid, pegaspargase, pentostatin, sipuleucel³, sizofiran, tamibarotene, temsirolimus, thalidomide, tretinoin, vismodegib, zoledronic acid, vorinostat;
   celecoxib, cilengitide, entinostat, etanidazole, ganetespib, idronoxil, iniparib, ixazomib, lonidamine, nimorazole, panobinostat, peretinoin, plitidepsin, pomalidomide, procodazol, ridaforolimus, tasquinimod, telotristat, thymalfasin, tirapazamine, tosedostat, trabedersen, ubenimex, valspodar, gendicine⁴, picibanil⁴, reolysin⁴, retaspimycin hydrochloride^{1,3}, trebananib^{2,3}, virulizin⁴, carfilzomib^{1,3}, endostatin⁴, immucothel⁴, belinostat³, MGN-1703⁴;
   **¹ Prop. INN (P**roposed **I**nternational **N**onproprietary **N**ame)
   **² Rec. INN (R**ecommended **I**nternational **N**onproprietary **N**ames)
   **³ USAN** (**U**nited **S**tates **A**dopted **N**ame)
   **⁴ no INN.**

The following abbreviations refer respectively to the definitions below:
aq (aqueous), h (hour), g (gram), L (liter), mg (milligram), MHz (Megahertz), min. (minute), mm (millimeter), mmol (millimole), mM (millimolar), m.p. (melting point), eq (equivalent), mL (milliliter), L (microliter), ACN (acetonitrile), AcOH (acetic acid), CDCl₃ (deuterated chloroform), CD₃OD (deuterated methanol), CH₃CN (acetonitrile), c-hex (cyclohexane), DCC (dicyclohexyl carbodiimide), DCM (dichloromethane), DIC (diisopropyl carbodiimide), DIEA (diisopropylethyl-amine), DMF (dimethylformamide), DMSO (dimethylsulfoxide), DMSO-d₆ (deuterated dimethylsulfoxide), EDC (1-(3-dimethyl-amino-propyl)-3-ethylcarbodiimide), ESI (Electro-spray ionization), EtOAc (ethyl acetate), Et₂O (diethyl ether), EtOH (ethanol), HATU (dimethylamino-([1,2,3]triazolo[4,5-b]pyridin-3-yloxy)-methylene]-dimethylammonium hexafluorophosphate), HPLC (High Performance Liquid Chromatography), i-PrOH (2-propanol), K₂CO₃ (potassium carbonate), LC (Liquid Chromatography), MeOH (methanol), MgSO₄ (magnesium sulfate), MS (mass spectrometry), MTBE (Methyl tert-butyl ether), NaHCOs (sodium bicarbonate), NaBH₄ (sodium borohydride), NMM (N-methyl morpholine), NMR (Nuclear Magnetic Resonance), PyBOP (benzotriazole-1-yl-oxy-tris-pyrrolidino-phosphonium hexafluorophosphate), RT (room temperature), Rt (retention time), SPE (solid phase extraction), TBTU (2-(1-H-benzotriazole-1-yl)-1,1,3,3-tetramethyluromium tetrafluoro borate), TEA (triethylamine), TFA (trifluoroacetic acid), THF (tetrahydrofuran), TLC (Thin Layer Chromatography), UV (Ultraviolet).

### Description of the in vitro assays

### Abbreviations:

GST = Glutathione-S-transferase
FRET= Fluorescence resonance energy transfer
HTRF^{®} = (homogenous time resolved fluorescence)
HEPES = 4-(2-hydroxyethyl)-1-piperazine ethanesulfonic acid buffer
DTT = Dithiothreitol
BSA = bovine serum albumin
CHAPS = detergent;
CHAPS = 3-[(3-cholamidopropyl)dimethylammonio]-1-propanesulfonate

Streptavidin-XLent^{®} is a high grade streptavidin-XL665 conjugate for which the coupling conditions have been optimized to yield a conjugate with enhanced performances for some assays, particularly those requiring high sensitivity.

### Measurement of cellular inhibition of tankyrase

Since Tankyrases have been described to modulate cellular level of Axin2 (Huang et al., 2009; Nature) the increase of Axin2 level is used as readout for determination of cellular inhibition of Tankyrases in a Luminex based assay.

Cells of the colon carcinoma cell line DLD1 are plated in 96 well plates with 1.5×10⁴ cells per well. Next day, cells are treated with a serial dilution of test compound in seven steps as triplicates with a final DMSO concentration of 0.3%. After 24 hours, cells are lysed in lysis buffer (20mM Tris/HCl pH 8.0, 150 mM NaCl, 1% NP40, 10% Glycerol) and lysates are cleared by centrifugation through a 96 well filter plate (0.65µm). Axin2 protein is isolated from cell lysates by incubation with a monoclonal anti-Axin2 antibody (R&D Systems #MAB6078) that is bound to fluorescent carboxybeads. Then, bound Axin2 is specifically detected with a polyclonal anti-Axin2 antibody (Cell Signaling #2151) and an appropriate PE-fluorescent secondary antibody. The amount of isolated Axin2 protein is determined in a Luminex²⁰⁰ machine (Luminex Corporation) according to the manufacturer's instruction by counting 100 events per well. Inhibition of Tankyrase by test compounds results in higher levels of Axin2 which directly correlates with an increase of detectable fluorescence. As controls cells are treated with solvent alone (neutral control) and with a Tankyrase reference inhibitor IWR-2 (3E-06 M) which refers as control for maximum increase of Axin2. For analysis, the obtained data are normalized against the untreated solvent control and fitted for determination of the EC₅₀ values using the Assay Explorer software (Accelrys).

### Description of the TNKS1 and TNKS2 ELISA assay

### Biochemical activity testing of TNKS 1 and 2: activity ELISA (Autoparsylation assay)

For analysis of autoparsylation activity of TNKS 1 and 2 an activity ELISA iss performed: In the first step GST tagged TNKS is captured on a Glutathione coated plate. Then the activity assay with biotinylated NAD is performed in the absence/presence of the compounds. During the enzymatic reaction GST tagged TNKS transfers biotinylated ADP-ribose to itself from biotinylated NAD as co-substrate. For the detection streptavidin-HRP conjugate is added that binds to the biotinylated TNKS and is thereby captured to the plates. The amount of biotinylated resp. autoparsylated TNKS is detected with a luminescence substrate for HRP. The level of the luminescence signal correlats directly with the amount of autoparsylated TNKS and therefore with activity of TNKS.

The acitivity ELISA is performed in 384 well Glutathione coated microtiter plates (Express capture Glutathione coated plate, Biocat, Heidelberg, Germany). The plates are pre-equilibrated with PBS. Then the plates are incubated with 50 µl 20 ng/well GST-tagged Tnks-1 (1023-1327 aa, prepared in-house), respectively GST-tagged Tnks-2 (873-1166 aa, prepared in-house) in assay buffer (50 mM HEPES, 4 mM Mg-chloride, 0.05 % Pluronic F-68, 2 mM DTT, pH 7.7) overnight at 4°C. The plates are washed 3 times with PBS-Tween-20. The wells are blocked by incubation at room temperature for 20 minutes with 50 µl blocking buffer (PBS, 0.05 % Tween-20, 0.5 % BSA). Afterwards the plates are washed 3 times with PBS-Tween-20. The enzymatic reaction is performed in 50 µl reaction solution (50 mM HEPES, 4 mM Mg-chloride, 0.05 % Pluronic F-68, 1.4 mM DTT, 0.5 % DMSO, pH 7.7) with10 µM bio-NAD (Biolog, Life science Inst., Bremen, Germany) as co-substrate in the absence or presence of the test compound (10 dilution concentrations) for 1 hour at 30°C. The reaction is stopped by 3 times washing with PBS-Tween-20. For the detection 50 µl of 20ng/µl Streptavidin, HRP conjugate (MoBiTec, Göttingen, Germany) in PBS/0.05%Tween-20/0.01%BSA are added and the plates are incubated for 30 minutes at room temperature. After three times washing with PBS-Tween-20 50 µl of SuperSignal ELISA Femto Maximum sensitivity substrate solution (ThermoFisherScientific (Pierce), Bonn, Germany) are added. Following a 1minute incubation at room temperature luminescence signals are measured with an Envision multimode reader (Perkin Elmer LAS Germany GmbH) at 700 nm. The full value used is the inhibitor-free reaction. The pharmacological zero value used is XAV-939 (Tocris) in a final concentration of 5 µM. The inhibitory values (IC50) are determined using either the program Symyx Assay Explorer^{®} or Condosseo^{®} from GeneData.

Above and below, all temperatures are indicated in°C. In the following examples, "conventional work-up" means: water is added if necessary, the pH is adjusted, if necessary, to values between 2 and 10, depending on the constitution of the end product, the mixture is extracted with ethyl acetate or dichloromethane, the phases are separated, the organic phase is dried over sodium sulfate and evaporated, and the residue is purified by chromatography on silica gel and/or by crystallisation. Rf values on silica gel; eluent: ethyl acetate/methanol 9:1.

### Chemicals

- Potassium phosphate buffer: 0.05 M potassium phosphate buffer pH 7.4 containing 1 mM MgCl₂
- NADPH (nicotinamide adenine dinucleotide phosphate): 22.5 mg NADPH-Na₄ in 1.8 mL potassium phosphate buffer
- Acetonitrile: 50 Vol% acetonitrile (1 volume acetonitrile, 1 volume water)
- DMSO: 20 Vol% DMSO in water
- Stock solution of 20 mg/mL human or mouse liver microsomes (protein)/mL in phosphate buffer

Stock solution of 10 mM compound in 100% DMSO
¹H NMR was recorded on Bruker DPX-300, DRX-400, AVII-400 or on a 500 MHz spectrometer, using residual signal of deuterated solvent as internal reference. Chemical shifts (δ) are reported in ppm relative to the residual solvent signal (δ = 2.49 ppm for ¹H NMR in DMSO-d₆). ¹H NMR data are reported as follows: chemical shift (multiplicity, coupling constants, and number of hydrogens). Multiplicity is abbreviated as follows: s (singlet), d (doublet), t (triplet), q (quartet), m (multiplet), br (broad).

### HPLC/MS conditions (A):

Gradient: A:B = 96:4 to 0:100 in 3.4 min; Flow rate: 2.40 mL/min
A: Water + formic acid (0.05 %); B: Acetonitrile + formic acid (0.04 %)
Column: Chromolith SpeedROD RP-18e, 50 × 4.6 mm²
Wavelength: 220 nm

### Synthesis of intermediates

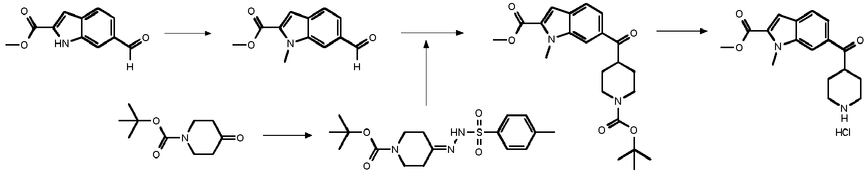

### A1: 1-Methyl-6-(piperidine-4-carbonyl)-1H-indole-2-carboxylic acid methyl ester hydrochloride

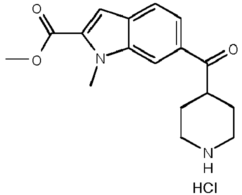

A1.1: 6-Formyl-1-methyl-1H-indole-2-carboxylic acid methyl ester 6-Formyl-1H-indole-2-carboxylic acid methyl ester (615.0 mg; 2.875 mmol) and cesium carbonate (1.89 g; 5.751 mmol) were suspended in acetonitrile (20.0 mL; 382.920 mmol). Iodomethane (255.7 µL; 4.025 mmol) was added, the vial was closed, and the mixture was heated to 60 °C for 14 h. The reaction was filtered over celite, washed with ethyl acetate and the filtrate was evaporated to dryness. The residue was purified by flash chromatography (CombiFlashRF 200). Yield: 571 mg (85%) pale yellow solid; LC/MS (A), Rt: 2.16 min; (M+H) 218.1.

A1.2: tert-Butyl 4-(p-tolylsulfonylhydrazono)piperidine-1-carboxylate 4-Methylbenzenesulfonohydrazide (5.10 g; 27.385 mmol) and 4-oxo-piperidine-1-carboxylic acid tert-butyl ester (5.46 g; 27.385 mmol) were suspended in dry methanol (50.0 mL). The mixture was stirred for 45 min at room temperature. The precipitate was filtered by suction, washed with a small amount of methanol and ethanol and then dried under vacuum at 50 °C for 1 h. Yield: 9.12 g (91%) colorless solid; LC/MS (A): Rt: 2.23 min; (M+H) 368.2.

### A1.3: 6-(1-tert-Butoxycarbonyl-piperidine-4-carbonyl)-1-methyl-1H-indole-2-carboxylic acid methyl ester

A1.1 (1.67 g; 7.603 mmol), A1.2 (3.00 g; 7.603 mmol), and cesium carbonate (4.95 g; 15.207 mmol) were suspended in dry 1,4-dioxane (30 mL). The mixture was heated to 110 °C and stirred for 14 h. The reaction mixture was cooled to room temperature, and dioxane was removed in vacuo. The residue was treated with water (120 mL) and extracted 3 times with ethyl acetate. The combined organic layers were washed with brine, dried with sodium sulfate, filtered by suction and evaporated to dryness. The resulting oil was purified by flash chromatography (CombiFlashRF 200). Yield: 1.45 g (48%) light yellow solid; LC/MS (A): Rt: 2.67 min; (M+H-t-Bu) 345.2.

### A1.4: 1-Methyl-6-(piperidine-4-carbonyl)-1H-indole-2-carboxylic acid methyl ester hydrochloride

A1.3 (1.45 g; 3.621 mmol) was dissolved in dry 1,4-dioxane (15.5 mL) and HCl solution (4M in dioxane; 7.24 mL; 28.967 mmol) was added. The clear solution was stirred at room temperature for 14 h. A suspension was formed, which was evaporated to dryness. Yield: 1.22 g (100%) yellow solid; LC/MS (A): Rt: 1.55 min; (M+H) 301.2.

### A2: 1-Methyl-5-(piperidine-4-carbonyl)-1H-indole-2-carboxylic acid ethyl ester hydrochloride

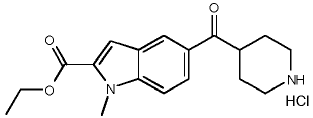

A2.1: 5-Formyl-1-methyl-1H-indole-2-carboxylic acid ethyl ester 5-Bromo-1-methyl-1H-indole-2-carboxylic acid ethyl ester (2.85 g; 10.102 mmol), palladium(II)-acetate (47% Pd; 45.4 mg; 0.202 mmol), CataCXium A (217.3 mg; 0.606 mmol) N,N,N',N'-tetramethylethylendiamine (885.1 mg; 7.617 mmol) were placed in an autoclave, toluene (67 mL) was added and the autoclave was pressurized with CO (3.7 bar) and heated at 100 °C for 14 h. The mixture was filtered over celite and the filtrate evaporated to dryness. The residue was purified by flash chromatography (CombiFlashRF 200). Yield: 2.17 g (94%) light brown oil; LC/MS (A): Rt: 2.27 min; (M+H) 232.1.

### A2.2: 5-(1-tert-Butoxycarbonyl-piperidine-4-carbonyl)-1-methyl-1H-indole-2-carboxylic acid ethyl ester

Preparation as described for intermediate A1.3. Yield: 1.71 g (45%) light yellow solid; LC/MS (A): Rt: 2.74 min; (M+H-t-Bu) 359.1.

### A2.3: 1-Methyl-5-(piperidine-4-carbonyl)-1H-indole-2-carboxylic acid ethyl ester hydrochloride

Preparation as described for intermediate A1.4. Yield: 1.41 g (98%) yellow solid; LC/MS (A): Rt: 1.58 min; (M+H) 315.1.

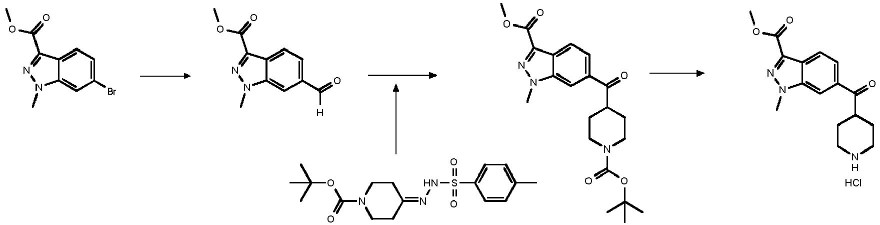

### A3: 1-Methyl-6-(piperidine-4-carbonyl)-1H-indazole-3-carboxylic acid methyl ester hydrochloride

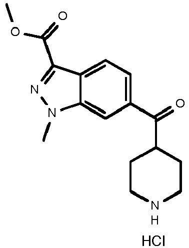

A3.1: 6-formyl-1-methyl-1H-indazole-3-carboxylic acid methyl ester Preparation as described for intermediate A2.1 using 6-bromo-1-methyl-1H-indazole-3-carboxylic acid methyl ester (449.0 mg; 1.669 mmol), palladium(II)-acetate (47% Pd; 5.6 mg; 0.025 mmol), CataCXium A (27.0 mg; 0.075 mmol) N,N,N',N'-tetramethylethylendiamine (126.0 mg; 1.084 mmol) in toluene (20 mL) with CO (4.8 bar) at 100 °C for 14 h. The mixture was filtered over celite and the filtrate evaporated to dryness. The residue was purified by flash chromatography (CombiFlashRF 200). Yield: 360 mg (99%) light yellow solid; LC/MS (A): Rt: 1.78 min; (M+H) 219.1.

### A3.2: 6-(1-tert-Butoxycarbonyl-piperidine-4-carbonyl)-1-methyl-1H-indazole-3-carboxylic acid methyl ester

A3.1 (360.0 mg; 1.643 mmol), A1.2 (600.0 mg; 1.633 mmol), and cesium carbonate (798.0 mg; 2.449 mmol) were suspended in dry 1,4-dioxane (10.0 mL) and the mixture was heated to 100 °C and stirred for 14 h. The reaction mixture was cooled to room temperature, quenched with water (50 mL) and extracted 3 times with ethyl acetate. The combined organic layers were washed with brine, dried with sodium sulfate, filtered by suction and evaporated to dryness. The oily residue was purified by chromatography (Companion RF; 50 g C18HP silica gel column). The collected fractions with product were combined and evaporated to an aqueous residue, which was rendered basic with saturated NaHCOs solution and extracted 3 times with ethyl acetate. The combined organic layers were washed with brine, dried with sodium sulfate, filtered by suction and evaporated to dryness. Yield: 190 mg (29%) light yellow foam; LC/MS (A): Rt: 2.40 min; (M+H-t-Bu) 346.1.

### A3.3: 1-Methyl-6-(piperidine-4-carbonyl)-1H-indazole-3-carboxylic acid methyl ester hydrochloride

Cleavage of the Boc-protecting group was performed as described for intermediate A1.4. Yield: 153 mg (96%) pale brown solid; LC/MS (A): Rt: 1.35 min; (M+H) 302.1.

### A4: 1-Methyl-5-(piperidine-4-carbonyl)-1H-indazole-3-carboxylic acid methyl ester hydrochloride

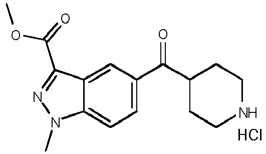

A4.1: 5-formyl-1-methyl-1H-indazole-3-carboxylic acid methyl ester Preparation as described for intermediate A3.1 using 5-bromo-1-methyl-1H-indazole-3-carboxylic acid methyl ester (955.5 mg; 3.551 mmol), palladium(II)-acetate (47% Pd; 12 mg; 0.053 mmol), CataCXium A (57.5 mg; 0.160 mmol) N,N,N',N'-tetramethylethylendiamine (269.0 mg; 2.306 mmol) in toluene (40 mL) with CO (6.3 bar) at 100 °C for 14 h. The reaction mixture was diluted with ethyl acetate (50 mL), washed once with water, 10% citric acid solution, saturated NaHCOs solution and brine, dried with sodium sulfate, filtered by suction and evaporated to dryness. The product was used in the next step without further purification. Yield: 699 mg (90%) yellow solid; LC/MS (A): Rt: 1.72 min; (M+H) 219.1.

### A4.2: 5-(1-tert-Butoxycarbonyl-piperidine-4-carbonyl)-1-methyl-1H-indazole-3-carboxylic acid methyl ester

Preparation was performed as described for intermediate A3.2. Yield: 273 mg (47%) yellow oil; LC/MS (A): Rt: 2.35 min; (M+H-Boc) 302.1.

### A4.3: 1-Methyl-5-(piperidine-4-carbonyl)-1H-indazole-3-carboxylic acid methyl ester hydrochloride

Cleavage of the Boc-protecting group was performed as described for intermediate A1.4. Yield: 230 mg (100%) light orange solid; LC/MS (A): Rt: 1.31 min; (M+H) 302.1.

### A5: (1-Methyl-1H-indazol-6-yl)-piperidin-4-yl-methanone dihydrochloride

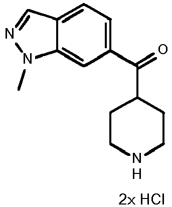

### A5.1: 1-Methyl-1H-indazole-6-carbaldehyde

Preparation as described for intermediate A3.1 using 6-bromo-1-methyl-1H-indazole (2.23 g; 10.571 mmol), palladium(II)-acetate (47% Pd; 43.0 mg; 0.192 mmol), CataCXium A (204.0 mg; 0.569 mmol) N,N,N',N'-tetramethyl-ethylendiamine (830.0 mg; 7.142 mmol) in toluene (60 mL) with CO (5.6 bar) at 100 °C for 7 h. Purification by flash chromatography (CombiFlashRF 200). Yield: 1.59 g (94%) light yellow solid; LC/MS (A): Rt: 1.66 min; (M+H) 161.1.

### A5.2: 4-(1-Methyl-1H-indazole-6-carbonyl)-piperidine-1-carboxylic acid tert-butyl ester

Preparation was performed as described for intermediate A3.2. Yield: 4.50 g (79%) light yellow solid; LC/MS (A): Rt: 2.38 min; (M+H) 344.2.

### A5.3: (1-Methyl-1H-indazol-6-yl)-piperidin-4-yl-methanone dihydrochloride

Cleavage of the Boc-protecting group was performed as described for intermediate A1.4. Yield: 3.43 g (97%) light yellow solid; LC/MS (A): Rt: 1.22 min; (M+H) 244.1.

### A6: (1-Methyl-1H-indazol-5-yl)-piperidin-4-yl-methanone hydrochloride

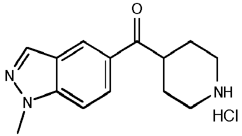

### A6.1: 4-(1-Methyl-1H-indazole-5-carbonyl)-piperidine-1-carboxylic acid tert-butyl ester

Preparation was performed as described for intermediate A3.2. Yield: 2.60 g (63%) yellow oil; LC/MS (A): Rt: 2.31 min; (M+H-t-Bu) 288.1.

### A6.2: (1-Methyl-1H-indazol-5-yl)-piperidin-4-yl-methanone hydrochloride

Cleavage of the Boc-protecting group was performed as described for intermediate A1.4. Yield: 2.04 g (96%) beige solid; LC/MS (A): Rt: 1.15 min; (M+H) 244.1.

### A7: (3-Methyl-3H-benzimidazol-5-yl)-piperidin-4-yl-methanone dihydrochloride

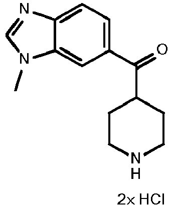

### A7.1: 4-(3-Methyl-3H-benzimidazole-5-carbonyl)-piperidine-1-carboxylic acid tert-butyl ester

Preparation was performed as described for intermediate A3.2. Yield: 452 mg (54%) yellow oil; LC/MS (A): Rt: 1.83 min; (M+H) 344.2.

### A7.2: (3-Methyl-3H-benzimidazol-5-yl)-piperidin-4-yl-methanone dihydrochloride

Cleavage of the Boc-protecting group was performed as described for intermediate A1.4. Yield: 428 mg (100%) colorless solid; LC/MS (A): Rt: 0.48 min; (M+H) 244.2.

### A8: (1-Methyl-1H-benzimidazol-5-yl)-piperidin-4-yl-methanone hydrochloride

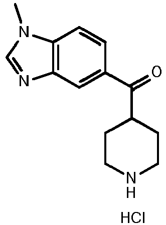

### A8.1: 4-(1-Methyl-1H-benzimidazole-5-carbonyl)-piperidine-1-carboxylic acid tert-butyl ester

Preparation was performed as described for intermediate A3.2. Yield: 597 mg (59%) yellow oil; LC/MS (A): Rt: 1.87-1.90 min; (M+H-t-Bu) 288.1.

### A8.2: (1-Methyl-1H-benzimidazol-5-yl)-piperidin-4-yl-methanone hydrochloride

Cleavage of the Boc-protecting group was performed as described for intermediate A1.4. Yield: 484 mg (100%) yellow solid; LC/MS (A): Rt: 0.39-0.64 min; (M+H) 244.2.

### A9: (3-Methyl-3H-benzotriazol-5-yl)-piperidin-4-yl-methanone hydrochloride

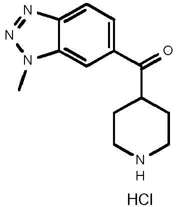

### A9.1: 3-Methyl-3H-benzotriazole-5-carbaldehyde

Preparation as described for intermediate A3.1 using 6-Bromo-1-methyl-1H-benzotriazole (7.70 g; 36.289 mmol), palladium(II)-acetate (47% Pd; 162.9 mg; 0.726 mmol), CataCXium A (650.6 mg; 1.814 mmol) N,N,N',N'-tetra-methylethylendiamine (2.74 g; 23.588 mmol) in toluene (100 mL) with CO (4.5 bar) at 80 °C for 16 h. Yield: 4.79 g (82%) brown solid; LC/MS (A): Rt: 1.37 min; (M+H) 162.0.

### A9.2: 4-(3-Methyl-3H-benzotriazole-5-carbonyl)-piperidine-1-carboxylic acid tert-butyl ester

Preparation was performed as described for intermediate A3.2. Yield: 11.12 g (83%) yellow oil; LC/MS (A): Rt: 2.20 min; (M+H) 345.0.

### A9.3: (3-Methyl-3H-benzotriazol-5-yl)-piperidin-4-yl-methanone hydrochloride

Cleavage of the Boc-protecting group was performed as described for intermediate A1.4. Yield: 9.1 g (100%) pale brown solid; LC/MS (A): Rt: 1.05 min; (M+H) 245.1.

### A10: 5-Methylamino-pyridine-2-carboxylic acid methyl ester hydrochloride

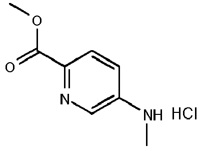

### A10.1: 5-(tert-Butoxycarbonyl-methyl-amino)-pyridine-2-carboxylic acid methyl ester

5-tert-Butoxycarbonylamino-pyridine-2-carboxylic acid methyl ester (282.0 mg; 1.118 mmol) and sodium hydride suspension (60% suspension in paraffin oil; 29.5 mg; 1.230 mmol) were stirred in DMF (3.0 mL) under nitrogen atmosphere at room temperature for 10 min. lodomethane (174.5 mg; 1.230 mmol) was added and the reaction mixture was stirred at room temperature for 2 h. Further sodium hydride suspension (60% suspension in paraffin oil; 16.1 mg; 0.671 mmol) and iodomethane (95.2 mg; 0.671 mmol) were added and the reaction mixture stirred at room temperature overnight. The mixture was diluted with water and extracted with ethyl acetate. The combined organic phases were dried over sodium sulfate, filtered and concentrated. The residue was dried under high vacuum. Yield: 152.0 mg (51%) pale-orange solid; LC/MS (A): Rt: 1.98 min; (M+H) 267.1.

A10.2: 5-Methylamino-pyridine-2-carboxylic acid methyl ester hydrochloride 5-(tert-Butoxycarbonyl-methyl-amino)-pyridine-2-carboxylic acid methyl ester (152.0 mg; 0.571 mmol) was dissolved in dry 1,4-dioxane (2.0 mL). A solution of HCl in dioxane (4 M, 1.43 mL; 5.708 mmol) was added and the mixture was stirred at room temperature for 14 h. The reaction mixture was evaporated to dryness. The residue was suspended in mtb-ether, filtered by suction and washed with mtb-ether. The solid was dried under vacuum at 50 °C. Yield: 115.5 mg (100%) pale-orange solid; LC/MS (A): Rt: 1.18 min; (M+H) 167.2.

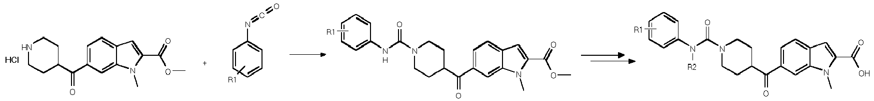

### Example 1: 6-{1-[(4-Fluoro-phenyl)-methyl-carbamoyl]-piperidine-4-carbonyl}-1-methyl-1H-indole-2-carboxylic acid ("C1")

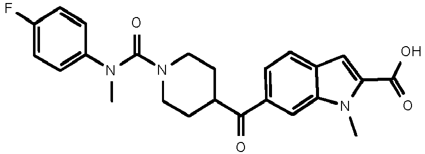

Step 1.1: 1-Methyl-6-(piperidine-4-carbonyl)-1H-indole-2-carboxylic acid methyl ester hydrochloride (intermediate A1, 188.7 mg; 0.560 mmol) was suspended in dichloromethane (2.5 mL) while stirring under argon atmosphere. N-Ethyldiisopropylamine (275.8 mg; 2.134 mmol) was added and the reaction mixture was stirred at room temperature for 5 min. 1-Fluoro-4-isocyanato-benzene (73.2 mg; 0.534 mmol) was added dropwise over a period of 1 min at room temperature and the solution was stirred at room temperature for 14 h. The reaction mixture was diluted with dichloromethane, washed twice with water, dried with sodium sulfate, filtered by suction and evaporated to dryness. Yield: 233 mg (100%) yellow foam; LC/MS (A): Rt: 2.39 min; (M+H) 438.2.

Step 1.2: 6-[1-(4-Fluoro-phenylcarbamoyl)-piperidine-4-carbonyl]-1-methyl-1H-indole-2-carboxylic acid methyl ester (116.1 mg; 0.265 mmol) and cesium carbonate (190.8 mg; 0.584 mmol) were suspended in DMF (1.5 mL). lodomethane (75.4 mg; 0.531 mmol) was added and the mixture was stirred at room temperature for 14 h. Further iodomethane (45.3 mg; 0.319 mmol) was added and the mixture was stirred at room temperature for 4 d. The reaction mixture was diluted with water (30 mL) and extracted with ethyl acetate (2 × 30 mL). The combined organic layers were washed with brine, dried over sodium sulfate, filtered, and concentrated in vacuo. The residue was purified by RP-flash chromatography (CombiFlashRF 200). The combined fractions were concentrated in vacuo, diluted with saturated aqueous NaHCOs-solution, and extracted with ethyl acetate (2 × 30 mL). The combined organic layers were washed with brine, dried over sodium sulfate, filtered, and concentrated in vacuo. Yield: 115.4 mg (96%) yellow oil; LC/MS (A): Rt: 2.53 min; (M+H) 452.2.

Step 1.3: 6-{1-[(4-Fluoro-phenyl)-methyl-carbamoyl]-piperidine-4-carbonyl}-1-methyl-1H-indole-2-carboxylic acid methyl ester (115.4 mg; 0.256 mmol) was dissolved in 1,4-dioxane (0.84 mL). While stirring sodium hydroxide solution (2 M; 383.5 µL; 0.767 mmol) was added and the reaction mixture was stirred at 80 °C for 2 h, cooled to room temperature and stirred for 14 h. The reaction mixture was diluted with water (2 mL) and acidified to pH 4 with 0.1 N HCl solution. A colorless precipitate was formed, which was filtered by suction, rinsed with water and dried under vacuum overnight. The crude product was purified by RP-flash chromatography (CombiFlashRF 200). The combined fractions were concentrated in vacuo, diluted with saturated aqueous NaHCOs-solution, and extracted with ethyl acetate (2 × 30 mL). The combined organic layers were washed with brine, dried over sodium sulfate, filtered, and concentrated in vacuo. Yield: 68 mg (61%) colorless solid; LC/MS (A): Rt: 2.20 min; (M+H) 438.1; ¹H NMR (500 MHz, DMSO-d₆) δ 13.20 (s, 1H), 8.23 (s, 1H), 7.76 (d, *J* = 8.4 Hz, 1H), 7.68 (dd, *J* = 8.5, 1.3 Hz, 1H), 7.26 (s, 1H), 7.24-7.16 (m, 4H), 4.12 (s, 3H), 3.80-3.67 (m, 3H), 3.08 (s, 3H), 2.83 (td, *J* = 13.0, 2.3 Hz, 2H), 1.72-1.63 (m, 2H), 1.36 (qd, *J* = 12.7, 4.0 Hz, 2H).

The following examples were prepared accordingly:

### Example 2: 6-{1-[Ethyl-(4-fluoro-phenyl)-carbamoyl]-piperidine-4-carbonyl}-1-methyl-1H-indole-2-carboxylic acid ("C2")

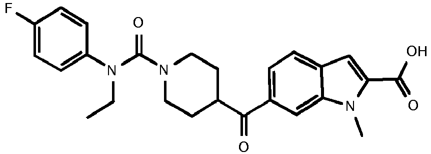

Yield: 41 mg (75%) beige solid; LC/MS (A): Rt: 2.31 min; (M+H) 452.2; ¹H NMR (500 MHz, DMSO-d₆) δ 13.19 (s, br, 1H), 8.20 (s, 1H), 7.74 (d, *J* = 8.4 Hz, 1H), 7.66 (dd, *J* = 8.5, 1.3 Hz, 1H), 7.23 (s, 1H), 7.22-7.15 (m, 4H), 4.10 (s, 3H), 3.76-3.63 (m, 3H), 3.60-3.51 (m, 2H), 2.77 (td, *J* = 13.0, 2.2 Hz, 2H), 1.68-1.59 (m, 2H), 1.31 (qd, *J* = 12.7, 3.9 Hz, 2H), 1.01 (t, *J* = 7.0 Hz, 3H).

### Example 3: 6-{1-[(4-Methoxy-phenyl)-methyl-carbamoyl]-piperidine-4-carbonyl}-1-methyl-1H-indole-2-carboxylic acid ("C3")

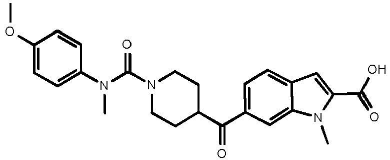

Yield: 87.5 (78%) beige solid; LC/MS (A): Rt: 2.17 min; (M+H) 450.2; ¹H NMR (500 MHz, DMSO-d₆) δ 13.21 (s, br, 1H), 8.23 (s, 1H), 7.76 (d, *J* = 8.4 Hz, 1H), 7.69 (dd, *J* = 8.5, 1.3 Hz, 1H), 7.26 (s, 1H), 7.10 (d, *J* = 8.9 Hz, 2H), 6.95 (d, *J* = 9.0 Hz, 2H), 4.12 (s, 3H), 3.82-3.74 (m, 5H), 3.70 (tt, *J* = 11.3, 3.0 Hz, 1H), 3.05 (s, 3H), 2.80 (td, *J* = 13.0, 2.1 Hz, 2H), 1.69-1.62 (m, 2H), 1.34 (qd, *J* = 12.7, 4.0 Hz, 2H).

### Example 4: 6-{1-[Ethyl-(4-methoxy-phenyl)-carbamoyl]-piperidine-4-carbonyl}-1-methyl-1H-indole-2-carboxylic acid ("C4")

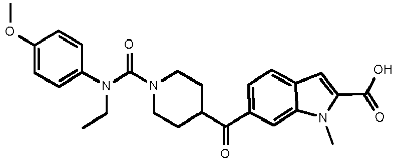

Yield: 78 mg (79%) beige solid; LC/MS (A): Rt: 2.26 min; (M+H) 464.2; ¹H NMR (500 MHz, DMSO-d₆) δ 13.17 (s, br, 1H), 8.20 (s, 1H), 7.73 (d, *J* = 8.4 Hz, 1H), 7.65 (dd, *J* = 8.5, 1.3 Hz, 1H), 7.24 (s, 1H), 7.06 (d, *J* = 8.9 Hz, 2H), 6.93 (d, *J* = 9.0 Hz, 2H), 4.10 (s, 3H), 3.78-3.69 (m, 5H), 3.65 (tt, *J* = 11.3, 3.5 Hz, 1H), 3.51 (q, *J* = 6.9 Hz, 2H), 2.74 (td, *J* = 13.0, 2.0 Hz, 2H), 1.61 (d, *J* = 10.9 Hz, 2H), 1.29 (qd, *J* = 12.7, 3.9 Hz, 2H), 0.99 (t, *J* = 7.0 Hz, 3H).

### Example 5: 5-{1-[(4-Fluoro-phenyl)-methyl-carbamoyl]-piperidine-4-carbonyl}-1-methyl-1H-indole-2-carboxylic acid ("C5")

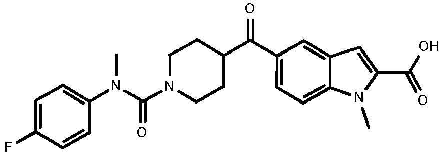

Step 5.1: 1-Methyl-5-(piperidine-4-carbonyl)-1H-indole-2-carboxylic acid ethyl ester hydrochloride (intermediate A2; 312.0 mg; 0.889 mmol) was suspended in dichloromethane (2.50 mL) while stirring under argon atmosphere. N-Ethyldiisopropylamine (459.5 mg; 3.555 mmol) was added and the reaction mixture was stirred at room temperature for 5 min. A colorless suspension was formed. 1-Fluoro-4-isocyanato-benzene (119.4 mg; 0.871 mmol) was added at room temperature and the solution was stirred at room temperature for 14 h. The reaction mixture was diluted with dichloromethane, washed twice with water, dried with sodium sulfate, filtered by suction and evaporated to dryness. The residue was purified by flash chromatography (CombiFlashRF 200). Yield: 391 mg (97%) light yellow foam; LC/MS (A): Rt: 2.45 min; (M+H) 452.2.

Step 5.2: 5-[1-(4-Fluoro-phenylcarbamoyl)-piperidine-4-carbonyl]-1-methyl-1H-indole-2-carboxylic acid ethyl ester (195.4 mg; 0.433 mmol) and cesium carbonate (310.2 mg; 0.952 mmol) were suspended in DMF (3.0 mL). lodomethane (122.8 mg; 0.865 mmol) was added and the mixture was stirred at room temperature for 14 h. Further iodomethane (73.7 mg; 0.519 mmol) was added and the mixture was stirred at room temperature for 14 h. The reaction mixture was diluted with (30 mL) and extracted with ethyl acetate (2 × 30 mL). The combined organic layers were washed with brine, dried over sodium sulfate, filtered, and concentrated in vacuo. The residue was purified by RP-flash chromatography (CombiFlashRF 200). The combined fractions were concentrated in vacuo, diluted with saturated aqueous NaHCOs-solution, and extracted with ethyl acetate (2 × 30 mL). The combined organic layers were washed with brine, dried over sodium sulfate, filtered, and concentrated in vacuo. Yield: 186 mg (92%) yellow oil; LC/MS (A): Rt: 2.59 min; (M+H) 466.2.

Step 5.3: 5-{1-[(4-Fluoro-phenyl)-methyl-carbamoyl]-piperidine-4-carbonyl}-1-methyl-1H-indole-2-carboxylic acid ethyl ester (186.0 mg; 0.399 mmol) was dissolved in 1,4-dioxane (0.25 mL). While stirring sodium hydroxide solution (2 M; 598.7 µL; 1.197 mmol) was added and the reaction mixture was stirred at 80 °C for 2 h, cooled to room temperature and stirred for 14 h. The reaction was diluted with water (2 mL) and acidified to pH 4 with 0.1 N HCl solution. A colorless precipitate was formed, which was filtered by suction, rinsed with water and dried under vacuum for 14 h. Yield: 130 mg (74%) beige solid; LC/MS (A): Rt: 2.17 min; (M+H) 438.2; ¹H NMR (400 MHz, DMSO-d₆) δ 13.10 (s, br, 1H), 8.43 (d, *J* = 1.2 Hz, 1H), 7.88 (dd, *J* = 8.9, 1.6 Hz, 1H), 7.64 (d, *J* = 9.0 Hz, 1H), 7.35 (s, 1H), 7.23-7.15 (m, 4H), 4.04 (s, 3H), 3.77-3.68 (m, 2H), 3.66-3.56 (m, 1H), 3.07 (s, 3H), 2.80 (td, *J* = 12.9, 2.0 Hz, 2H), 1.69-1.59 (m, 2H), 1.35 (qd, *J* = 12.6, 3.9 Hz, 2H).

The following examples were prepared accordingly:

### Example 6: 5-{1-[Ethyl-(4-fluoro-phenyl)-carbamoyl]-piperidine-4-carbonyl}-1-methyl-1H-indole-2-carboxylic acid ("C6")

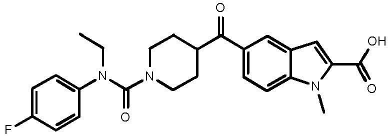

Yield: 133 mg (85%) pale beige solid; LC/MS (A): Rt: 2.27 min; (M+H) 452.2; ¹H NMR (500 MHz, DMSO-d₆) δ 13.14 (s, 1H), 8.44 (d, *J* = 1.3 Hz, 1H), 7.90 (dd, *J* = 8.9, 1.7 Hz, 1H), 7.67 (d, *J* = 9.0 Hz, 1H), 7.37 (s, 1H), 7.26-7.13 (m, 4H), 4.06 (s, 3H), 3.73 (d, *J* = 13.1 Hz, 2H), 3.65-3.50 (m, 3H), 2.78 (td, *J* = 13.0, 2.2 Hz, 2H), 1.63 (d, *J* = 11.0 Hz, 2H), 1.32 (qd, *J* = 12.7, 4.0 Hz, 2H), 1.03 (t, *J* = 7.0 Hz, 3H).

### Example 7: 5-{1-[(4-Methoxy-phenyl)-methyl-carbamoyl]-piperidine-4-carbonyl}-1-methyl-1H-indole-2-carboxylic acid ("C7")

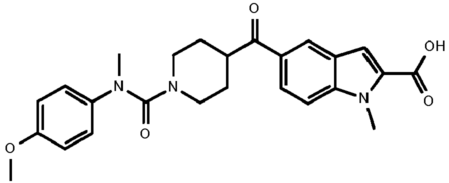

Yield: 97 mg (79%) beige solid; LC/MS (A): Rt: 2.13 min; (M+H) 450.2; ¹H NMR (500 MHz, DMSO-d₆) δ 13.13 (s, br, 1H), 8.43 (d, *J* = 1.2 Hz, 1H), 7.89 (dd, *J* = 8.9, 1.6 Hz, 1H), 7.65 (d, *J* = 9.0 Hz, 1H), 7.36 (s, 1H), 7.09 (d, *J* = 8.9 Hz, 2H), 6.94 (d, *J* = 9.0 Hz, 2H), 4.05 (s, 3H), 3.80-3.70 (m, 5H), 3.59 (tt, *J* = 11.2, 3.4 Hz, 1H), 3.04 (s, 3H), 2.76 (td, *J* = 12.9, 2.0 Hz, 2H), 1.66-1.59 (m, 2H), 1.33 (qd, *J* = 12.7, 3.9 Hz, 2H).

### Example 8: 5-{1-[Ethyl-(4-methoxy-phenyl)-carbamoyl]-piperidine-4-carbonyl}-1-methyl-1H-indole-2-carboxylic acid ("C8")

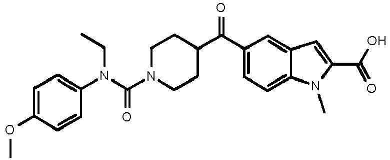

Yield: 69 mg (73%) beige solid; LC/MS (A): Rt: 2.23 min; (M+H) 464.2; ¹H NMR (500 MHz, DMSO-d₆) δ 13.12 (s, br, 1H), 8.42 (d, *J* = 1.3 Hz, 1H), 7.88 (dd, *J* = 8.9, 1.6 Hz, 1H), 7.65 (d, *J* = 9.0 Hz, 1H), 7.36 (s, 1H), 7.07 (d, *J* = 8.9 Hz, 2H), 6.94 (d, *J* = 9.0 Hz, 2H), 4.05 (s, 3H), 3.78-3.67 (m, 5H), 3.62-3.47 (m, 3H), 2.73 (td, *J* = 13.0, 2.0 Hz, 2H), 1.65-1.54 (m, 2H), 1.30 (qd, *J* = 12.7, 3.9 Hz, 2H), 1.00 (t, *J* = 7.0 Hz, 3H).

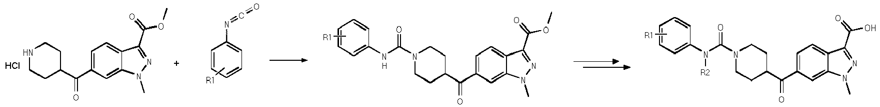

### Example 9: 6-{1-[(4-Fluoro-phenyl)-methyl-carbamoyl]-piperidine-4-carbonyl}-1-methyl-1H-indazole-3-carboxylic acid ("C9")

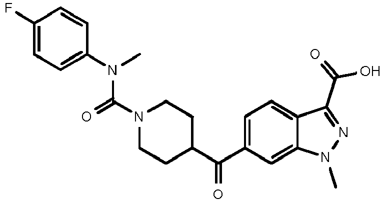

Step 9.1: 1-Methyl-6-(piperidine-4-carbonyl)-1H-indazole-3-carboxylic acid methyl ester hydrochloride (intermediate A3; 140.0 mg; 0.414 mmol) was suspended in dichloromethane (3.0 mL) while stirring under argon atmosphere. N-Ethyldiisopropylamine (141.5 mg; 1.095 mmol) was added and the mixture was stirred for 5 min at room temperature. 4-Fluorophenyl isocyanate (62.5 mg; 0.456 mmol) was added and the mixture was stirred at room temperature for 14 h. The reaction mixture was diluted with dichloromethane, washed twice with water, dried with sodium sulfate, filtered by suction and evaporated to dryness. The residue was purified by flash chromatography (CombiFlashRF 200). Yield: 172 mg (95%) colorless oil; LC/MS (A): Rt: 2.15 min; (M+H) 439.1.

Step 9.2: 6-[1-(4-Fluoro-phenylcarbamoyl)-piperidine-4-carbonyl]-1-methyl-1H-indazole-3-carboxylic acid methyl ester (86.5 mg; 0.197 mmol) and cesium carbonate (128.6 mg; 0.395 mmol) were suspended in DMF (2.0 mL). Iodomethane (56.0 mg; 0.395 mmol) was added and the mixture was stirred at room temperature for 14 h. Further iodomethane (56.0 mg; 0.395 mmol) was added and the reaction was stirred at 50 °C for 3 d. The reaction mixture was evaporated to dryness, the residue dissolved in dichloromethane and filtered through a syringe filter. The filtrate was evaporated to dryness and the residue purified by RP-flash chromatography (CombiFlashRF 200). The combined fractions were concentrated in vacuo, diluted with saturated aqueous NaHCOs-solution, and extracted with dichloromethane (3 x 40 mL). The combined organic layers were dried over sodium sulfate, filtered, and concentrated in vacuo. Yield: 40 mg (45%) yellow oil; LC/MS (A): Rt: 2.26 min; (M+H) 453.1.

Step 9.3: 6-{1-[(4-Fluoro-phenyl)-methyl-carbamoyl]-piperidine-4-carbonyl}-1-methyl-1H-indazole-3-carboxylic acid methyl ester (40.0 mg; 0.088 mmol) was suspended in 1,4-dioxane (0.3 mL). Sodium hydroxide solution (2 M; 111.7 µL; 0.223 mmol) was added and the reaction mixture was stirred at room temperature for 14 h. Further sodium hydroxide solution (2 M; 111.7 µL; 0.223 mmol) was added and the reaction mixture was stirred at room temperature overnight. The reaction was diluted with water, acidified to pH 3-4 with 0.1 N HCl solution. The precipiated solid was filtered by suction and dried under high vacuum. Yield: 10 mg (26%) colorless solid; LC/MS (A): Rt: 2.02 min; (M+H) 439.1; ¹H NMR (500 MHz, DMSO-d₆) δ 13.17 (s, 1H), 8.45-8.43 (m, 1H), 8.14-8.12 (m, 1H), 7.82 (dd, *J* = 8.6, 1.3 Hz, 1H), 7.23-7.17 (m, 4H), 4.24 (s, 3H), 3.77-3.68 (m, 3H), 3.07 (s, 3H), 2.81 (td, *J* = 12.8, 2.5 Hz, 2H), 1.72-1.66 (m, 2H), 1.38-1.29 (m, 2H).

The following example was prepared accordingly:

### Example 10: 6-{1-[Ethyl-(4-fluoro-phenyl)-carbamoyl]-piperidine-4-carbonyl}-1-methyl-1H-indazole-3-carboxylic acid ("C10")

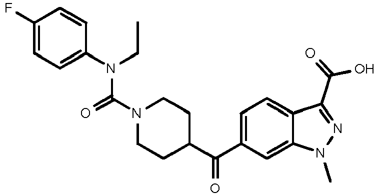

Yield: 15 mg (92%) colorless solid; LC/MS (A): Rt: 2.13 min; (M+H) 453.1; 1H NMR (500 MHz, DMSO-d6) δ 13.29-13.11 (m, 1H), 8.44-8.43 (m, 1H), 8.14-8.11 (m, 1H), 7.82 (dd, *J* = 8.6, 1.3 Hz, 1H), 7.24-7.16 (m, 4H), 4.24 (s, 3H), 3.74-3.66 (m, 3H), 3.56 (q, *J* = 6.9 Hz, 2H), 2.77 (td, *J* = 12.8, 2.5 Hz, 2H), 1.69-1.63 (m, 2H), 1.34-1.25 (m, 2H), 1.01 (t, *J* = 7.0 Hz, 3H).

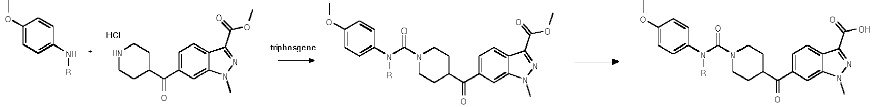

### Example 11: 6-{1-[(4-Methoxy-phenyl)-methyl-carbamoyl]-piperidine-4-carbonyl}-1-methyl-1H-indazole-3-carboxylic acid ("C11")

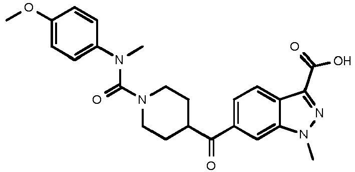

Step 11.1: 4-Methoxy-N-methylaniline (39.2 mg; 0.286 mmol) and ditrichloromethyl carbonate (29.7 mg; 0.100 mmol) were dissolved in dry methanol (2.0 mL). N-Ethyldiisopropylamine (145.8 µL; 0.857 mmol) was added and the mixture was stirred at room temperature for 15 min. The solution was added dropwise to a suspension of 1-methyl-6-(piperidine-4-carbonyl)-1H-indazole-3-carboxylic acid methyl ester hydrochloride (intermediate A3; 96.6 mg; 0.286 mmol) at room temperature. The mixture was heated to 50 °C and stirred for 14 h. The mixture was diluted with water and extracted with mtb-ether. The combined organic layers were washed with brine, dried with sodium sulfate, filtered by suction and evaporated to dryness. The oily residue was purified by flash chromatography (Companion RF; 24 g SI50 silica gel column). Yield: 55 mg (41%) pale brown solid; LC/MS (A): Rt: 2.24 min; (M+H) 465.2.

Step 11.2: 6-{1-[(4-Methoxy-phenyl)-methyl-carbamoyl]-piperidine-4-carbonyl}-1-methyl-1H-indazole-3-carboxylic acid methyl ester (54.8 mg; 0.118 mmol) was dissolved in 1,4-dioxane (0.24 mL). Sodium hydroxide solution (2 M; 117.5 µl; 0.235 mmol) was added and the solution was stirred at room temperature for 5 h. Hydrochloric acid (2 M; 117.5 µl; 0.235 mmol) was added to the reaction mixture. The solution was diluted with water and extracted with ethyl acetate. The combined organic layers were washed with brine, dried with sodium sulfate, filtered by suction and evaporated to dryness. The crude product was suspended in a small amount of acetonitrile, filtered by suction and washed with a small amount of mtb-ether. The solid was dried under high vacuum at room temperature. Yield: 41 mg (78%) colorless solid; LC/MS (A): Rt: 1.98 min; (M+H) 451.2; ¹H NMR (500 MHz, DMSO-d₆) δ 13.15 (s, 1H), 8.44-8.42 (m, 1H), 8.14-8.11 (m, 1H), 7.81 (dd, *J* = 8.6, 1.4 Hz, 1H), 7.11-7.06 (m, 2H), 6.95-6.91 (m, 2H), 4.23 (s, 3H), 3.78-3.72 (m, 5H), 3.72-3.65 (m, 1H), 3.03 (s, 3H), 2.81-2.73 (m, 2H), 1.69-1.63 (m, 2H), 1.36-1.26 (m, 2H).

The following example was prepared accordingly:

### Example 12: 6-{1-[Ethyl-(4-methoxy-phenyl)-carbamoyl]-piperidine-4-carbonyl}-1-methyl-1H-indazole-3-carboxylic acid ("C12")

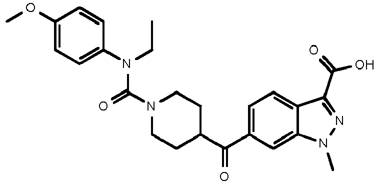

Yield: 10 mg (59%) colorless solid; LC/MS (A): Rt: 2.09 min; (M+H) 465.2; ¹H NMR (500 MHz, DMSO-d₆) δ 13.15 (s, 1H), 8.45-8.39 (m, 1H), 8.12 (d, *J* = 8.6 Hz, 1H), 7.80 (dd, *J* = 8.6, 1.3 Hz, 1H), 7.10-7.03 (m, 2H), 6.97-6.90 (m, 2H), 4.23 (s, 3H), 3.76-3.69 (m, 5H), 3.69-3.62 (m, 1H), 3.51 (q, *J* = 6.9 Hz, 2H), 2.73 (td, *J* = 12.8, 2.5 Hz, 2H), 1.68-1.58 (m, 2H), 1.34-1.21 (m, 2H), 0.99 (t, *J* = 6.9 Hz, 3H).

### Example 13: 5-{1-[(4-Fluoro-phenyl)-methyl-carbamoyl]-piperidine-4-carbonyl}-1-methyl-1H-indazole-3-carboxylic acid ("C13")

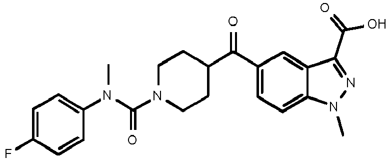

Step 13.1: 1-Methyl-5-(piperidine-4-carbonyl)-1H-indazole-3-carboxylic acid methyl ester hydrochloride (intermediate A4; 230.0 mg; 0.681 mmol) was suspended in dichloromethane (3.9 mL) while stirring under argon atmosphere. N-Ethyldiisopropylamine (281.7 mg; 2.179 mmol) was added and the mixture was stirred for 5 min at room temperature. 4-Fluorophenyl isocyanate (102.7 mg; 0.749 mmol) was added and the mixture was stirred room temperature overnight. The reaction mixture was diluted with dichloromethane, washed twice with water, dried with sodium sulfate, filtered by suction and evaporated to dryness. The residue was purified by flash chromatography (CombiFlashRF 200). Yield: 290 mg (100%) yellow foam; LC/MS (A): Rt: 2.11 min; (M+H) 439.1.

Step 13.2: 5-[1-(4-Fluoro-phenylcarbamoyl)-piperidine-4-carbonyl]-1-methyl-1H-indazole-3-carboxylic acid methyl ester (157.0 mg; 0.358 mmol) and cesium carbonate (233.3 mg; 0.716 mmol) were suspended in DMF (3.0 mL). Iodomethane (101.6 mg; 0.716 mmol) was added and the mixture was stirred at room temperature for 14 h. Further iodomethane (101.6 mg; 0.716 mmol) was added and the reaction mixture was stirred at 50 °C for 24 h. The reaction mixture was evaporated to dryness. The residue was dissolved in dichloromethane, filtered through a syringe filter and the filtrate evaporated to dryness. The residue was purified by RP-flash chromatography (CombiFlashRF 200). The combined fractions were concentrated in vacuo, diluted with saturated aqueous NaHCOs-solution, and extracted with ethyl acetate (3 x 40 mL). The combined organic layers were dried over sodium sulfate, filtered, and concentrated in vacuo. Yield: 85.4 mg (53%) colorless solid; LC/MS (A): Rt: 2.22 min; (M+H) 453.1.

Step 13.3: 5-{1-[(4-Fluoro-phenyl)-methyl-carbamoyl]-piperidine-4-carbonyl}-1-methyl-1H-indazole-3-carboxylic acid methyl ester (85.4 mg; 0.189 mmol) was suspended in 1,4-dioxane (0.6 mL). Sodium hydroxide solution (2 M; 235.8 µl; 0.472 mmol) was added and the reaction mixture was stirred at room temperature for 14 h. The reaction was diluted with water, acidified to pH 3-4 with 0.1 N HCl solution. Brine solution was added to the aqueous phase and the mixture was extracted with ethyl acetate (3 x 20 mL). The combined organic layers were washed with brine, dried over sodium sulfate, filtered, and concentrated in vacuo. The residue was freeze-dried. Yield: 33 mg (40%) colorless solid; LC/MS (A): Rt: 2.02 min; (M+H) 439.2; ¹H NMR (400 MHz, DMSO-d₆) δ 13.70-12.68 (m, 1H), 8.67-8.65 (m, 1H), 8.03 (dd, *J* = 9.0, 1.7 Hz, 1H), 7.86-7.83 (m, 1H), 7.23-7.17 (m, 4H), 4.17 (s, 3H), 3.76-3.68 (m, 2H), 3.66-3.57 (m, 1H), 3.07 (s, 3H), 2.86-2.78 (m, 2H), 1.70-1.62 (m, 2H), 1.41-1.29 (m, 2H).

The following examples were prepared accordingly:

### Example 14: 5-{1-[Ethyl-(4-fluoro-phenyl)-carbamoyl]-piperidine-4-carbonyl}-1-methyl-1H-indazole-3-carboxylic acid ("C14")

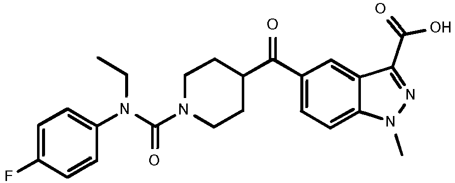

Yield: 45 mg (57%) colorless solid; LC/MS (A): Rt: 2.11 min; (M+H) 453.2; ¹H NMR (500 MHz, DMSO-d₆) δ 13.44-13.16 (m, 1H), 8.66-8.64 (m, 1H), 8.03 (dd, *J* = 9.0, 1.6 Hz, 1H), 7.86 (d, *J=* 8.9 Hz, 1H), 7.23-7.16 (m, 4H), 4.17 (s, 3H), 3.72-3.66 (m, 2H), 3.62-3.52 (m, 3H), 2.81-2.73 (m, 2H), 1.66-1.60 (m, 2H), 1.34-1.25 (m, 2H), 1.00 (t, *J* = 7.0 Hz, 3H).

### Example 15: 5-{1-[(4-Methoxy-phenyl)-methyl-carbamoyl]-piperidine-4-carbonyl}-1-methyl-1H-indazole-3-carboxylic acid ("C15")

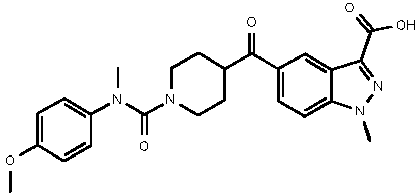

Yield: 29 mg (53%) pale-yellow solid; LC/MS (A): Rt: 1.96 min; (M+H) 451.2; ¹H NMR (500 MHz, DMSO-d₆) δ 13.23 (m, 1H), 8.66-8.64 (m, 1H), 8.03 (dd, *J* = 9.0, 1.6 Hz, 1H), 7.87-7.84 (m, 1H), 7.10-7.06 (m, 2H), 6.95-6.91 (m, 2H), 4.17 (s, 3H), 3.76-3.70 (m, 5H), 3.62-3.55 (m, 1H), 3.03 (s, 3H), 2.80-2.73 (m, 2H), 1.65-1.59 (m, 2H), 1.36-1.26 (m, 2H).

### Example 16: 5-{1-[Ethyl-(4-methoxy-phenyl)-carbamoyl]-piperidine-4-carbonyl}-1-methyl-1H-indazole-3-carboxylic acid ("C16")

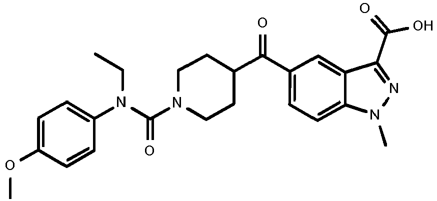

Yield: 27 mg (63%) pale-yellow solid; LC/MS (A): Rt: 2.06 min; (M+H) 465.2; ¹H NMR (500 MHz, DMSO-d₆) δ 13.47-13.11 (m, 1H), 8.65-8.64 (m, 1H), 8.02 (dd, *J* = 9.0, 1.6 Hz, 1H), 7.85 (d, *J* = 8.9 Hz, 1H), 7.08-7.04 (m, 2H), 6.95-6.91 (m, 2H), 4.17 (s, 3H), 3.74 (s, 3H), 3.73-3.68 (m, 2H), 3.60-3.53 (m, 1H), 3.50 (q, *J* = 7.0 Hz, 2H), 2.76-2.70 (m, 2H), 1.63-1.57 (m, 2H), 1.33-1.23 (m, 2H), 0.98 (t, *J=* 7.0 Hz, 3H).

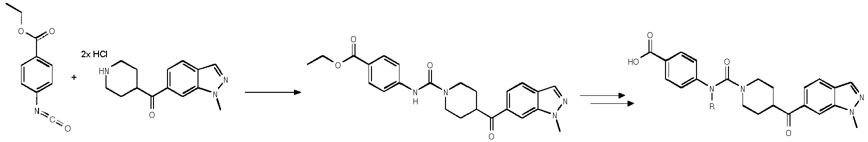

### Example 17: 4-{Methyl-[4-(1-methyl-1H-indazole-6-carbonyl)-piperidine-1-carbonyl]-amino}-benzoic acid ("C17")

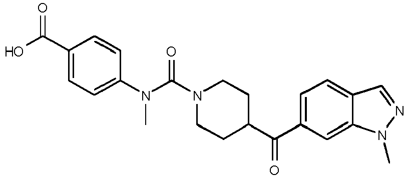

Step 17.1: (1-Methyl-1H-indazol-6-yl)-piperidin-4-yl-methanone dihydrochloride (intermediate A5; 237.9 mg; 0.752 mmol) was suspended in dichloromethane (5.0 mL). N-Ethyldiisopropylamine (353.3 mg; 2.733 mmol) was added and the reaction mixture was stirred at room temperature for 5 min. Ethyl 4-isocyanatobenzoate (130.6 mg; 0.683 mmol) was added at room temperature and reaction mixture was stirred at room temperature for 14 h. The reaction mixture was diluted with dichloromethane and washed twice with water. The organic phase was dried over sodium sulfate, filtered by suction and evaporated to dryness. Yield: 212 mg (71%) off-white solid; LC/MS (A): Rt: 2.24 min; (M+H) 435.2.

Step 17.2: 4-{[4-(1-Methyl-1H-indazole-6-carbonyl)-piperidine-1-carbonyl]-amino}-benzoic acid ethyl ester (118.4 mg; 0.272 mmol) was dissolved in DMF (5.0 mL), cesium carbonate (221.9 mg; 0.681 mmol) and iodomethane (77.3 mg; 0.545 mmol) was added while stirring at room temperature and the reaction mixture was stirred for 14 h. The reaction mixture was diluted with water and extracted with ethyl acetate. The combined organic layers were washed with brine, dried over sodium sulfate, filtered by suction and evaporated to dryness. The oily residue was purified by using flash chromatography (Companion RF; 24g Si50 silica gel column). Yield: 119 mg (97%) colorless oil; LC/MS (A): Rt: 2.30 min; (M+H) 449.2.

Step 17.3: 4-{Methyl-[4-(1-methyl-1H-indazole-6-carbonyl)-piperidine-1-carbonyl]-amino}-benzoic acid ethyl ester (239.0 mg; 0.533 mmol) was dissolved in 1,4-dioxane (3.0 mL). Sodium hydroxide solution (2 M; 1.87 mL; 3.730 mmol) was added and the reaction mixture was stirred at room temperature for 14 h and at 40 °C for 2 h. The reaction was diluted with water, acidified to pH 3-4 with 0.1 N HCl solution. Brine solution was added to the aqueous phase and the mixture was extracted with ethyl acetate. The combined organic layers were washed with brine, dried over sodium sulfate, filtered, and concentrated in vacuo. The residue was purified by flash chromatography (Companion RF; 24g Si50 silica gel column) and freeze-dried. Yield: 128 mg (57%) colorless solid; LC/MS (A): Rt: 1.92 min; (M+H) 421.1; ¹H NMR (400 MHz, DMSO-d₆) δ 12.80-12.55 (m, 1H), 8.34-8.33 (m, 1H), 8.14 (d, *J* = 0.9 Hz, 1H), 7.92-7.87 (m, 2H), 7.86-7.83 (m, 1H), 7.67 (dd, *J* = 8.5, 1.3 Hz, 1H), 7.17-7.13 (m, 2H), 4.14 (s, 3H), 3.84-3.72 (m, 3H), 3.17 (s, 3H), 2.95 (td, *J* = 12.8, 2.6 Hz, 2H), 1.80-1.73 (m, 2H), 1.52-1.40 (m, 2H).

The following examples were prepared accordingly:

### Example 18: 4-{Ethyl-[4-(1-methyl-1H-indazole-6-carbonyl)-piperidine-1-carbonyl]-amino}-benzoic acid ("C18")

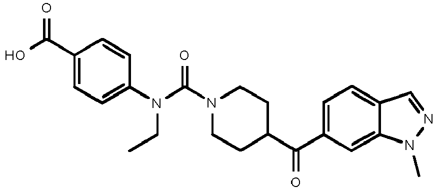

Yield: 19 mg (62%) colorless powder; LC/MS (A): Rt: 1.99 min; (M+H) 435.2; ¹H NMR (400 MHz, DMSO-d₆) δ 12.71 (s, 1H), 8.32 (s, 1H), 8.13 (s, 1H), 7.95-7.87 (m, 2H), 7.84 (d, *J=* 8.5 Hz, 1H), 7.66 (d, *J=* 8.6 Hz, 1H), 7.19-7.10 (m, 2H), 4.13 (s, 3H), 3.82-3.61 (m, 5H), 2.87 (t, *J* = 12.7 Hz, 2H), 1.77-1.67 (m, 2H), 1.48-1.33 (m, 2H), 1.13-1.03 (m, 3H).

### Example 19: 3-{Methyl-[4-(1-methyl-1H-indazole-6-carbonyl)-piperidine-1-carbonyl]-amino}-benzoic acid ("C19")

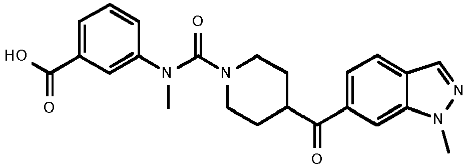

Yield: 95 mg (98%) colorless solid; LC/MS (A): Rt: 1.94 min; (M+H) 421.2; ¹H NMR (400 MHz, DMSO-d₆) δ 13.20-12.88 (m, 1H), 8.34-8.31 (m, 1H), 8.15-8.12 (m, 1H), 7.83 (d, *J=* 8.5 Hz, 1H), 7.68-7.62 (m, 3H), 7.48 (t, *J=* 7.8 Hz, 1H), 7.41-7.37 (m, 1H), 4.13 (s, 3H), 3.81-3.68 (m, 3H), 3.14 (s, 3H), 2.91-2.82 (m, 2H), 1.74-1.66 (m, 2H), 1.43-1.31 (m, 2H).

### Example 20: 3-{Ethyl-[4-(1-methyl-1H-indazole-6-carbonyl)-piperidine-1-carbonyl]-amino}-benzoic acid ("C20")

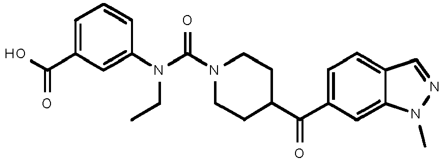

Yield: 56 mg (89%) colorless solid; LC/MS (A): Rt: 2.02 min; (M+H) 435.2; ¹H NMR (400 MHz, DMSO-d₆) δ 13.99-11.89 (m, 1H), 8.31 (s, 1H), 8.13 (s, 1H), 7.83 (d, *J=* 8.5 Hz, 1H), 7.70-7.60 (m, 3H), 7.49 (t, *J* = 7.8 Hz, 1H), 7.41-7.36 (m, 1H), 4.13 (s, 3H), 3.77-3.65 (m, 3H), 3.62 (q, *J=* 7.0 Hz, 2H), 2.85-2.75 (m, 2H), 1.70-1.62 (m, 2H), 1.37-1.26 (m, 2H), 1.04 (t, *J* = 6.9 Hz, 3H).

### Example 21: 5-{Methyl-[4-(1-methyl-1H-indazole-6-carbonyl)-piperidine-1-carbonyl]-amino}-pyridine-2-carboxylic acid ("C21")

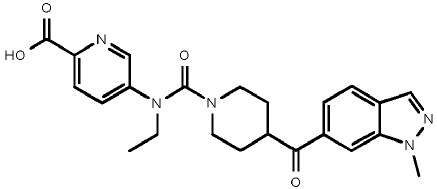

Step 21.1: 5-Methylamino-pyridine-2-carboxylic acid methyl ester hydrochloride (intermediate A10; 115.5 mg; 0.570 mmol) and ditrichloromethyl carbonate (84.6 mg; 0.285 mmol) were suspended in dry dichloromethane (6.0 mL). N-Ethyldiisopropylamine (484.7 µL; 2.850 mmol) was added and the mixture was stirred at room temperature for 30 min. The mixture was added dropwise to a solution of (1-methyl-1H-indazol-6-yl)-piperidin-4-yl-methanone hydrochloride (intermediate A5 as hydrochloride; 131.8 mg; 0.456 mmol) in dry methanol (2.0 mL) at room temperature and stirred at room temperature for 1 h. The mixture was diluted with dichloromethane, washed with water, dried over sodium sulfate, filtered by suction and evaporated to dryness. The oily residue was purified by prep. HPLC (Agilent 1260; Waters SunFire C18 5µm 30x150 mm column). Yield: 54 mg (22%) colorless foam; LC/MS (A): Rt: 1.92 min; (M+H) 436.2.

Step 21.2: 5-{Methyl-[4-(1-methyl-1H-indazole-6-carbonyl)-piperidine-1-carbonyl]-amino}-pyridine-2-carboxylic acid methyl ester (54.0 mg; 0.124 mmol) was dissolved in 1,4-dioxane (0.5 mL). Sodium hydroxide solution (2 M; 0.124 mL; 0.248 mmol) was added and the mixture stirred at 60 °C for 1 h. The reaction mixture was cooled to room temperature, HCl solution (2 M; 0.124 mL; 0.248 mmol) was added and the aqueous mixture evaporated to dryness. The residue was purified by flash chromatography (Companion RF; 12 g Si50 silica gel column) and freeze-dried. Yield: 23 mg colorless solid; LC/MS (A): Rt: 1.70 min; (M+H) 422.1; ¹H NMR (400 MHz, DMSO-d₆) δ 13.17-12.56 (m, 1H), 8.44 (d, *J* = 2.7 Hz, 1H), 8.36-8.33 (m, 1H), 8.15-8.12 (m, 1H), 8.00 (d, *J* = 8.6 Hz, 1H), 7.85 (d, *J* = 8.5 Hz, 1H), 7.68 (dd, *J* = 8.5, 1.3 Hz, 1H), 7.60 (dd, *J* = 8.6, 2.8 Hz, 1H), 4.15 (s, 3H), 3.86-3.75 (m, 3H), 3.22 (s, 3H), 3.06-2.97 (m, 2H), 1.85-1.76 (m, 2H), 1.57-1.44 (m, 2H).

### Example 22: 4-{Methyl-[4-(1-methyl-1H-indazole-5-carbonyl)-piperidine-1-carbonyl]-amino}-benzoic acid ("C22")

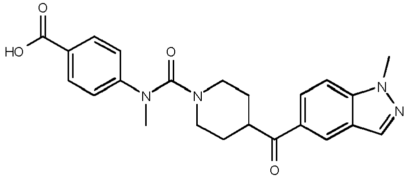

Step 22.1: (1-Methyl-1H-indazol-5-yl)-piperidin-4-yl-methanone hydrochloride (intermediate A6; 150.8 mg; 0.539 mmol) was suspended in dichloromethane (5.0 mL). N-Ethyldiisopropylamine (253.3 mg; 1.960 mmol) was added and the reaction mixture was stirred at room temperature for 5 min. Ethyl 4-isocyanatobenzoate (93.7 mg; 0.490 mmol) was added dropwise at room temperature and the suspension stirred at room temperature for 14 h. The reaction mixture was diluted with dichloromethane, washed twice with water, dried over sodium sulfate, filtered by suction, and evaporated to dryness. The residue was purified by prep. HPLC (Agilent 1260 HPLC; Waters SunFire C18 5µm 30x150 mm column) and freeze-dried. Yield: 65 mg (31%) colorless solid; LC/MS (A): Rt: 2.18 min; (M+H) 435.2.

Step 22.2: 4-{[4-(1-Methyl-1H-indazole-5-carbonyl)-piperidine-1-carbonyl]-amino}-benzoic acid ethyl ester (32.0 mg; 0.074 mmol) was dissolved in DMF (3.0 mL), cesium carbonate (52.8 mg; 0.162 mmol) and iodomethane (20.9 mg; 0.147 mmol) was added at room temperature and the reaction mixture was stirred for 14 h at 50 °C. Further iodomethane (10.5 mg; 0.074 mmol) and cesium carbonate (24.0 mg; 0.074 mmol) were added and the reaction mixture stirred for 14 h at 55 °C. The reaction mixture was evaporated, the residue purified by prep. HPLC (Agilent 1260 HPLC; Waters SunFire C18 5µm 30x150 mm column) and freeze-dried. Yield: 20.5 mg (62%) colorless oil; LC/MS (A): Rt: 2.25 min; (M+H) 449.2.

Step 22.3: 4-{Methyl-[4-(1-methyl-1H-indazole-5-carbonyl)-piperidine-1-carbonyl]-amino}-benzoic acid ethyl ester (20.5 mg; 0.046 mmol) was dissolved in 1,4-dioxane (0.16 mL). Sodium hydroxide solution (2 M; 0.057 mL; 0.114 mmol) was added and the reaction mixture was stirred room temperature for 14 h. The reaction mixture was treated with 0.1 M HCl solution (0.23 mL; 0.229 mmol). A precipitate was formed, which was filtered by suction, washed with water and dried under high vacuum for 14 h. Yield: 18 mg (94%) colorless solid; LC/MS (A): Rt: 1.88 min; (M+H) 421.2; ¹H NMR (500 MHz, DMSO-d₆) δ 12.70-12.64 (m, 1H), 8.57-8.55 (m, 1H), 8.23-8.22 (m, 1H), 7.96 (dd, *J* = 8.9, 1.6 Hz, 1H), 7.91-7.87 (m, 2H), 7.71 (d, *J=* 8.9 Hz, 1H), 7.16-7.12 (m, 2H), 4.07 (s, 3H), 3.83-3.77 (m, 2H), 3.73-3.66 (m, 1H), 3.16 (s, 3H), 2.97-2.90 (m, 2H), 1.76-1.70 (m, 2H), 1.51-1.41 (m, 2H).

The following examples were prepared accordingly:

### Example 23: 4-{Ethyl-[4-(1-methyl-1H-indazole-5-carbonyl)-piperidine-1-carbonyl]-amino}-benzoic acid ("C23")

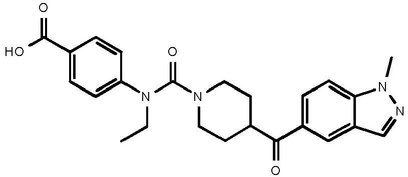

Yield: 26 mg (74%) colorless solid; LC/MS (A): Rt: 1.96 min; (M+H) 435.2; ¹H NMR (500 MHz, DMSO-d₆) δ 12.76-12.66 (m, 1H), 8.56-8.54 (m, 1H), 8.23-8.21 (m, 1H), 7.94 (dd, *J* = 8.9, 1.6 Hz, 1H), 7.92-7.88 (m, 2H), 7.70 (d, *J* = 8.9 Hz, 1H), 7.16-7.12 (m, 2H), 4.07 (s, 3H), 3.79-3.73 (m, 2H), 3.70-3.63 (m, 3H), 2.90-2.82 (m, 2H), 1.72-1.66 (m, 2H), 1.45-1.35 (m, 2H), 1.08 (t, *J* = 7.0 Hz, 3H).

### Example 24: 3-{Methyl-[4-(1-methyl-1H-indazole-5-carbonyl)-piperidine-1-carbonyl]-amino}-benzoic acid ("C24")

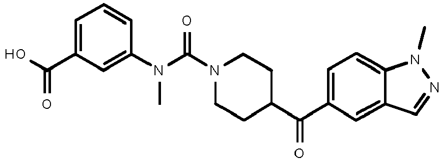

Yield: 440 mg (78%) colorless solid; LC/MS (A): Rt: 1.89 min; (M+H) 421.1; ¹H NMR (400 MHz, DMSO-d₆) δ 13.18-12.88 (m, 1H), 8.56-8.53 (m, 1H), 8.22-8.21 (m, 1H), 7.94 (dd, J = 8.9, 1.7 Hz, 1H), 7.70 (d, *J=* 8.9 Hz, 1H), 7.68-7.62 (m, 2H), 7.48 (t, *J=* 7.8 Hz, 1H), 7.40-7.36 (m, 1H), 4.07 (s, 3H), 3.81-3.73 (m, 2H), 3.70-3.60 (m, 1H), 3.14 (s, 3H), 2.90-2.80 (m, 2H), 1.72-1.63 (m, 2H), 1.44-1.31 (m, 2H).

### Example 25: 3-{Ethyl-[4-(1-methyl-1H-indazole-5-carbonyl)-piperidine-1-carbonyl]-amino}-benzoic acid ("C25")

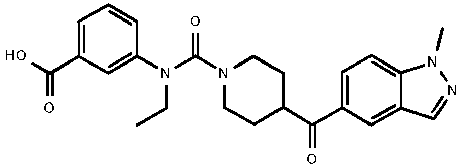

Yield: 96 mg (82%) colorless solid; LC/MS (A): Rt: 1.97 min; (M+H) 435.2; ¹H NMR (500 MHz, DMSO-d₆) δ 13.04 (s, 1H), 8.54-8.51 (m, 1H), 8.20 (d, *J* = 0.9 Hz, 1H), 7.93 (dd, *J* = 8.9, 1.6 Hz, 1H), 7.71-7.66 (m, 2H), 7.61 (t, *J* = 2.0 Hz, 1H), 7.49 (t, *J* = 7.8 Hz, 1H), 7.39-7.36 (m, 1H), 4.07 (s, 3H), 3.76-3.70 (m, 2H), 3.65-3.58 (m, 3H), 2.78 (td, *J* = 12.8, 2.5 Hz, 2H), 1.66-1.59 (m, 2H), 1.36-1.27 (m, 2H), 1.05 (t, *J* = 7.0 Hz, 3H).

### Example 26: 4-{Methyl-[4-(3-methyl-3H-benzoimidazole-5-carbonyl)-piperidine-1-carbonyl]-amino}-benzoic acid ("C26")

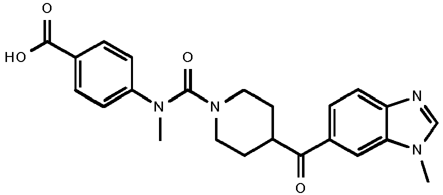

Step 26.1: (3-Methyl-3H-benzoimidazol-5-yl)-piperidin-4-yl-methanone dihydrochloride (intermediate A7; 176.5 mg; 0.558 mmol) was suspended in dry dichloromethane (3.0 mL) while stirring under argon atmosphere. N-Ethyldiisopropylamine (264.1 µL; 1.522 mmol) was added and the reaction mixture was stirred at room temperature for 5 min. A colorless suspension was formed. Ethyl 4-isocyanatobenzoate (100.0 mg; 0.507 mmol) dissolved in dry dichloromethane (2.0 mL) was added dropwise at room temperature and the mixture stirred at room temperature for 15 min. The reaction mixture was diluted with dichloro-methane, washed twice with water, dried with sodium sulfate, filtered by suction and evaporated to dryness. The oily residue was purified by flash chromatography (Companion RF; 12 g Si50 silica gel column). Yield: 77.3 mg (35%) colorless oil; LC/MS (A): Rt: 1.79 min; (M+H) 435.2.

Step 26.2: 4-{[4-(3-Methyl-3H-benzoimidazole-5-carbonyl)-piperidine-1-carbonyl]-amino}-benzoic acid ethyl ester (118.0 mg; 0.272 mmol) and cesium carbonate (177.0 mg; 0.543 mmol) were suspended in DMF (2.0 mL). Iodomethane (25.4 µL; 0.407 mmol) was added and the mixture was stirred at room temperature for 14 h. The reaction mixture was diluted with water and extracted with ethyl acetate. The combined organic layers were washed with brine, dried with sodium sulfate, filtered by suction and evaporated to dryness. The oily residue was purified prep. HPLC (Agilent 1260 HPLC; Waters SunFire C18 5µm 30×150 mm column). Yield: 78.5 mg (64%) light green oil; LC/MS (A): Rt: 1.85 min; (M+H) 449.2 .

Step 26.3: 4-{Methyl-[4-(3-methyl-3H-benzoimidazole-5-carbonyl)-piperidine-1-carbonyl]-amino}-benzoic acid ethyl ester (78.3 mg; 0.175 mmol) was suspended in 1,4-dioxane (0.350 mL). Sodium hydroxide solution (2 M; 0.175 mL; 0.349 mmol) was added and the mixture was heated at 60 °C for 1.5 h. The reaction mixture was cooled to room temperature and hydrochloric acid solution (2 M; 0.175 mL; 0.349 mmol) was added. A precipitate was formed, which was filtered by suction, washed with water, a small amount of acetonitrile and mtb-ether and then dried under vacuum at 60 °C for 14 h. Yield: 65 mg (89%) colorless solid; LC/MS (A): Rt: 1.49 min; (M+H) 421.2; ¹H NMR (500 MHz, DMSO-d₆) δ 12.68 (s, 1H), 8.38 (s, 1H), 8.26 (d, *J* = 1.2 Hz, 1H), 7.93-7.88 (m, 2H), 7.86 (dd, *J* = 8.5, 1.6 Hz, 1H), 7.73 (d, *J* = 8.5 Hz, 1H), 7.19-7.11 (m, 2H), 3.92 (s, 3H), 3.84-3.78 (m, 2H), 3.75 (tt, *J* = 11.3, 3.6 Hz, 1H), 3.17 (s, 3H), 2.96 (td, *J* = 13.0, 2.3 Hz, 2H), 1.81-1.72 (m, 2H), 1.48 (qd, *J=* 12.8, 4.0 Hz, 2H).

The following examples were prepared accordingly:

### Example 27: 4-{Ethyl-[4-(3-methyl-3H-benzimidazole-5-carbonyl)-piperidine-1-carbonyl]-amino}-benzoic acid ("C27")

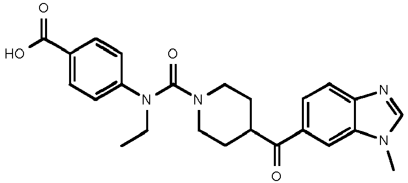

Yield: 42 mg (76%) colorless solid; LC/MS (A): Rt: 1.58 min; (M+H) 435.2; ¹H NMR (500 MHz, DMSO-d₆) δ 12.72 (s, 1H), 8.38 (s, 1H), 8.25 (d, *J* = 1.2 Hz, 1H), 7.91 (d, *J* = 8.7 Hz, 2H), 7.85 (dd, *J* = 8.5, 1.6 Hz, 1H), 7.72 (d, *J* = 8.5 Hz, 1H), 7.16 (d, *J* = 8.8 Hz, 2H), 3.92 (s, 3H), 3.85-3.75 (m, 2H), 3.75-3.64 (m, 3H), 2.88 (td, *J* = 13.0, 2.1 Hz, 2H), 1.76-1.67 (m, 2H), 1.42 (qd, *J* = 12.8, 4.0 Hz, 2H), 1.09 (t, *J* = 7.0 Hz, 3H).

### Example 28: 3-{Methyl-[4-(3-methyl-3H-benzimidazole-5-carbonyl)-piperidine-1-carbonyl]-amino}-benzoic acid ("C28")

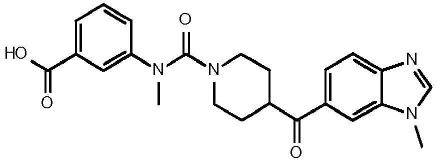

Yield: 21 mg (69%) colorless solid; LC/MS (A): Rt: 1.57 min; (M+H) 421.2; ¹H NMR (400 MHz, DMSO-d₆) δ 13.59-12.39 (m, 1H), 8.41-8.27 (m, 2H), 7.88 (d, *J* = 8.6 Hz, 1H), 7.75-7.59 (m, 3H), 7.44 (t, *J* = 7.7 Hz, 1H), 7.35-7.28 (m, 1H), 3.87 (s, 3H), 3.80-3.63 (m, 3H), 3.12 (s, 3H), 2.92-2.80 (m, 2H), 1.71-1.61 (m, 2H), 1.43-1.30 (m, 2H).

### Example 29: 3-{Ethyl-[4-(3-methyl-3H-benzimidazole-5-carbonyl)-piperidine-1-carbonyl]-amino}-benzoic acid ("C29")

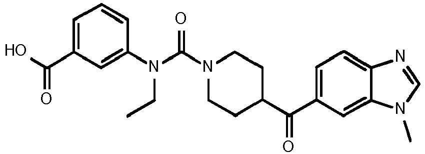

Yield: 18 mg (65%) colorless solid; LC/MS (A): Rt: 1.64 min; (M+H) 435.2; ¹H NMR (400 MHz, DMSO-d₆) δ 9.07 (s, 1H), 8.37-8.35 (m, 1H), 8.06-8.02 (m, 1H), 7.88 (d, *J* = 8.6 Hz, 1H), 7.69-7.65 (m, 1H), 7.62-7.60 (m, 1H), 7.49 (t, *J =* 7.8 Hz, 1H), 7.40-7.36 (m, 1H), 3.98 (s, 3H), 3.75-3.65 (m, 3H), 3.62 (q, *J =* 7.0 Hz, 2H), 2.85-2.76 (m, 2H), 1.67-1.60 (m, 2H), 1.36-1.24 (m, 2H), 1.04 (t, *J* = 7.0 Hz, 3H).

### Example 30: 4-{Methyl-[4-(1-methyl-1H-benzimidazole-5-carbonyl)-piperidine-1-carbonyl]-amino}-benzoic acid ("C30")

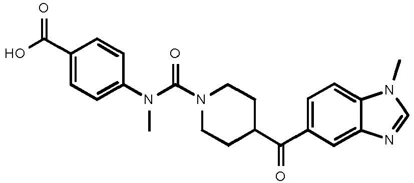

Step 30.1: (1-Methyl-1H-benzimidazol-5-yl)-piperidin-4-yl-methanone hydrochloride (intermediate A8; 200.0 mg; 0.715 mmol) was suspended in dry dichloromethane (3.0 mL) while stirring under argon atmosphere. N-Ethyldiisopropylamine (184.8 mg; 1.430 mmol) was added and the mixture was stirred for 5 min at room temperature. Ethyl 4-isocyanatobenzoate (136.7 mg; 0.715 mmol) was added and the mixture was stirred for 30 min at room temperature. Further ethyl 4-isocyanatobenzoate (205.0 mg; 1.072 mmol) and dry dichloromethane (2.0 mL) were added and the mixture was stirred at room temperature for 14 h. The reaction mixture was diluted with dichloromethane, washed with water, dried with sodium sulfate, filtered by suction and evaporated to dryness. The oily residue was purified by flash chromatography (Companion RF; 40 g Si50 silica gel column). Yield: 266 mg (86%) colorless foam; LC/MS (A): Rt: 1.84 min; (M+H) 435.2.

Step 30.2: 4-{[4-(1-Methyl-1H-benzoimidazole-5-carbonyl)-piperidine-1-carbonyl]-amino}-benzoic acid ethyl ester (135.4 mg; 0.312 mmol) and cesium carbonate (203.0 mg; 0.623 mmol) were suspended in DMF (2.0 mL). Iodomethane (88.4 mg; 0.623 mmol) was added and the mixture was stirred at room temperature for 14 h. The reaction mixture was diluted with water and extracted with ethyl acetate. The combined organic layers were washed with brine, dried with sodium sulfate, filtered by suction and evaporated to dryness. The oily residue was purified by prep. HPLC (Agilent 1100 HPLC; Merck Chromolith prep RP-18e 100-25 column). The combined fractions were evaporated to an aqueous residue, which was rendered basic with saturated NaHCOs solution and extracted with ethyl acetate. The combined organic layers were washed with brine, dried with sodium sulfate, filtered and evaporated to dryness. Yield: 64.9 mg (46%) pale-yellow foam; LC/MS (A): Rt: 1.91 min; (M+H) 449.2.

Step 30.3: 4-{Methyl-[4-(1-methyl-1H-benzoimidazole-5-carbonyl)-piperidine-1-carbonyl]-amino}-benzoic acid ethyl ester (64.9 mg; 0.145 mmol) was dissolved in 1,4-dioxane (0.3 mL). Sodium hydroxide solution (2 M; 0.145 mL; 0.289 mmol) was added and the mixture was stirred to 60 °C for 1 h. The reaction mixture was cooled to room temperature and hydrochloric acid (2 M; 0.145 mL; 0.289 mmol) was added. A precipitate was formed, which was filtered by suction, washed with a small amount of water, acetonitrile and mtb-ether and then dried under vacuum at 60 °C for 14 h. Yield: 49 mg (81%) colorless solid; LC/MS (A): Rt: 1.54 min; (M+H) 421.2; ¹H NMR (500 MHz, DMSO-d₆) δ 12.66 (s, 1H), 8.38 (d, *J* = 1.5 Hz, 1H), 8.33 (s, 1H), 7.92-7.87 (m, 3H), 7.67 (d, *J=* 8.5 Hz, 1H), 7.16-7.12 (m, 2H), 3.87 (s, 3H), 3.82-3.71 (m, 3H), 3.16 (s, 3H), 3.00-2.92 (m, 2H), 1.76-1.69 (m, 2H), 1.51-1.41 (m, 2H).

The following examples were prepared accordingly:

### Example 31: 4-{Ethyl-[4-(1-methyl-1H-benzimidazole-5-carbonyl)-piperidine-1-carbonyl]-amino}-benzoic acid ("C31")

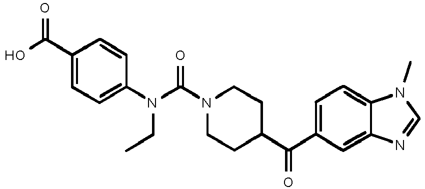

Yield: 58 mg (84%) colorless solid; LC/MS (A): Rt: 1.62 min; (M+H) 435.2; ¹H NMR (500 MHz, DMSO-d₆) δ 12.70 (s, 1H), 8.36 (d, *J* = 1.5 Hz, 1H), 8.32 (s, 1H), 7.92-7.87 (m, 3H), 7.66 (d, *J=* 8.5 Hz, 1H), 7.16-7.12 (m, 2H), 3.87 (s, 3H), 3.79-3.73 (m, 2H), 3.73-3.64 (m, 3H), 2.93-2.85 (m, 2H), 1.71-1.65 (m, 2H), 1.45-1.35 (m, 2H), 1.08 (t, *J* = 7.0 Hz, 3H).

### Example 32: 3-{Methyl-[4-(1-methyl-1H-benzimidazole-5-carbonyl)-piperidine-1-carbonyl]-amino}-benzoic acid ("C32")

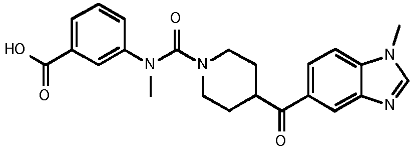

Yield: 18 mg (69%) colorless solid; LC/MS (A): Rt: 1.56 min; (M+H) 421.1; ¹H NMR (500 MHz, DMSO-d₆) δ 13.09-12.93 (m, 1H), 8.36-8.34 (m, 2H), 7.89 (dd, *J* = 8.5, 1.6 Hz, 1H), 7.68-7.62 (m, 3H), 7.48 (t, *J* = 7.8 Hz, 1H), 7.40-7.36 (m, 1H), 3.87 (s, 3H), 3.78-3.73 (m, 2H), 3.73-3.66 (m, 1H), 3.13 (s, 3H), 2.91-2.84 (m, 2H), 1.70-1.63 (m, 2H), 1.42-1.32 (m, 2H).

### Example 33: 3-{Ethyl-[4-(1-methyl-1H-benzimidazole-5-carbonyl)-piperidine-1-carbonyl]-amino}-benzoic acid ("C33")

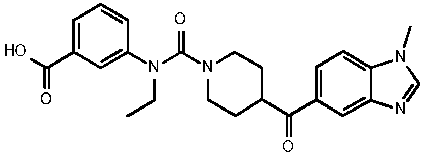

Yield: 47 mg (75%) colorless solid; LC/MS (A): Rt: 1.64 min; (M+H) 435.2; ¹H NMR (500 MHz, DMSO-d₆) δ 13.15-12.94 (m, 1H), 8.34-8.33 (m, 1H), 8.32 (s, 1H), 7.87 (dd, *J* = 8.6, 1.6 Hz, 1H), 7.69-7.66 (m, 1H), 7.65 (d, *J* = 8.6 Hz, 1H), 7.63-7.61 (m, 1H), 7.49 (t, *J=* 7.8 Hz, 1H), 7.39-7.36 (m, 1H), 3.86 (s, 3H), 3.75-3.69 (m, 2H), 3.69-3.60 (m, 3H), 2.85-2.78 (m, 2H), 1.65-1.59 (m, 2H), 1.36-1.26 (m, 2H), 1.05 (t, *J* = 7.0 Hz, 3H).

### Example 34: 4-{Methyl-[4-(3-methyl-3H-benzotriazole-5-carbonyl)-piperidine-1-carbonyl]-amino}-benzoic acid ("C34")

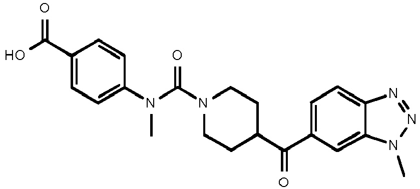

Step 34.1: (3-Methyl-3H-benzotriazol-5-yl)-piperidin-4-yl-methanone hydrochloride (intermediate A9; 236.2 mg; 0.841 mmol) was suspended in dichloromethane (5.0 mL). N-Ethyldiisopropylamine (0.52 mL; 3.059 mmol) was added and the reaction mixture was stirred at room temperature for 5 min. Ethyl 4-isocyanatobenzoate (146.2 mg; 0.765 mmol) was added dropwise at room temperature and the reaction mixture stirred at room temperature for 14 h. The reaction mixture was diluted with dichloromethane and washed water, dried over sodium sulfate, filtered by suction, and evaporated to dryness. The residue was purified by RP-flash chromatography. Yield: 137.2 mg (41%) off-white solid; LC/MS (A): Rt: 2.13 min; (M+H) 436.2.

Step 34.2: 4-{[4-(3-Methyl-3H-benzotriazole-5-carbonyl)-piperidine-1-carbonyl]-amino}-benzoic acid ethyl ester (68.6 mg; 0.158 mmol) was dissolved in DMF (4.0 mL), cesium carbonate (154.0 mg; 0.473 mmol) and iodomethane (67.1 mg; 0.473 mmol) was added and the reaction mixture stirred at room temperature for 14 h. The reaction mixture was diluted with water and extracted with ethyl acetate. The combined organic layers were washed once with brine, dried over sodium sulfate, filtered by suction and evaporated to dryness. The oily residue was purified by using RP-flash chromatography (Companion RF200). Yield: 34.9 mg (49%) colorless oil; LC/MS (A): Rt: 2.20 min; (M+H) 450.2.

Step 34.3: 4-{Methyl-[4-(3-methyl-3H-benzotriazole-5-carbonyl)-piperidine-1-carbonyl]-amino}-benzoic acid ethyl ester (34.9 mg; 0.078 mmol) was dissolved in 1,4-dioxane (0.5 mL). Sodium hydroxide solution (2 M; 0.078 mL; 0.155 mmol) was added and the mixture was stirred at 50 °C for 1 h and at room temperature for 14 h. Water and hydrochloric acid solution (2 M; 0.116 mL; 0.233 mmol) was added to the reaction mixture. A precipitate was formed, which was filtered by suction and dried under high vacuum for 14 h. Yield: 17 mg (52%) colorless solid; LC/MS (A): Rt: 1.80 min; (M+H) 422.2; ¹H NMR (400 MHz, DMSO-d₆) δ 12.67 (s, 1H), 8.59-8.56 (m, 1H), 8.11 (d, *J* = 8.8 Hz, 1H), 7.95-7.87 (m, 3H), 7.18-7.12 (m, 2H), 4.40 (s, 3H), 3.84-3.72 (m, 3H), 3.17 (s, 3H), 2.99-2.89 (m, 2H), 1.82-1.74 (m, 2H), 1.52-1.40 (m, 2H).

The following example was prepared accordingly:

### Example 35: 4-{Ethyl-[4-(3-methyl-3H-benzotriazole-5-carbonyl)-piperidine-1-carbonyl]-amino}-benzoic acid ("C35")

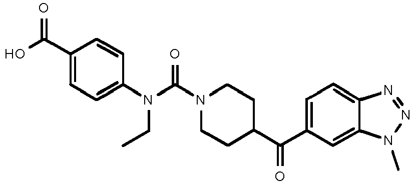

Yield: 17 mg (44%) colorless solid; LC/MS (A): Rt: 1.88 min; (M+H) 436.2; ¹H NMR (500 MHz, DMSO-d₆) δ 12.82-12.58 (m, 1H), 8.58-8.54 (m, 1H), 8.11 (d, *J=* 8.7 Hz, 1H), 7.94-7.88 (m, 3H), 7.17-7.13 (m, 2H), 4.39 (s, 3H), 3.80-3.63 (m, 5H), 2.91-2.83 (m, 2H), 1.77-1.70 (m, 2H), 1.45-1.35 (m, 2H), 1.08 (t, *J* = 7.0 Hz, 3H).

### Pharmacological data

**Table 1 Inhibition of tankyrases of representative compounds of the formula I**

| Compound No. | EC₅₀ [M] TNKS cellular assay | Compound No. | EC₅₀ [M] TNKS cellular assay |
|---|---|---|---|
| "C1" | 2.10 E-07 | "C21" | 1.50 E-05 |
| "C2" | 1.90 E-08 | "C22" | 3.46 E-07 |
| "C3" | 1.30 E-07 | "C23" | 7.50 E-09 |
| "C4" | 9.90 E-09 | "C24" | 6.40 E-07 |
| "C5" | 7.40 E-08 | "C25" | 4.87 E-08 |
| "C6" | 5.90 E-09 | "C26" | 1.10 E-06 |
| "C7" | 6.31 E-08 | "C27" | 2.90 E-07 |
| "C8" | 7.10 E-09 | "C28" | |
| "C9" | 4.90 E-06 | "C29" | 4.75 E-06 |
| "C10" | 4.60 E-07 | "C30" | 6.00 E-06 |
| "C11" | 3.90 E-06 | "C31" | 3.69 E-07 |
| "C12" | 3.00 E-07 | "C32" | |
| "C13" | | "C33" | 3.60 E-06 |
| "C14" | 9.40 E-06 | "C34" | 3.20 E-06 |
| "C15" | 7.10 E-06 | "C35" | 3.10 E-07 |
| "C16" | 9.70 E-07 | | |
| "C17" | 2.07 E-07 | | |
| "C18" | 1.28 E-08 | | |
| "C19" | 1.40 E-06 | | |
| "C20" | 1.93 E-07 | | |

The compounds shown in Table 1 are particularly preferred compounds according to the invention.

**Table 2 Inhibition of tankyrases of representative compounds of the formula I**

| Compound No. | IC₅₀ [M] TNKS1 ELISA | IC₅₀ [M] TN KS2 ELISA |
|---|---|---|
| "C1" | 3.50 E-09 | 1.10 E-09 |
| "C2" | 6.70 E-10 | 4.90 E-10 |
| "C3" | 1.80 E-09 | 2.70 E-09 |
| "C4" | 3.60 E-10 | 2.60 E-10 |
| "C5" | 1.00 E-09 | 1.40 E-09 |
| "C6" | 4.10 E-10 | 1.40 E-09 |
| "C7" | 1.30 E-09 | 1.30 E-09 |
| "C8" | 2.60 E-10 | 4.40 E-10 |
| "C9" | 3.29 E-09 | 1.66 E-09 |
| "C10" | 7.44 E-10 | 4.32 E-10 |
| "C11" | 1.40 E-09 | 7.40 E-10 |
| "C12" | 5.20 E-10 | 2.50 E-10 |
| "C13" | 1.15 E-07 | 4.99 E-08 |
| "C14" | 1.24 E-08 | 5.91 E-09 |
| "C15" | 4.30 E-08 | 1.90 E-08 |
| "C16" | 9.70 E-09 | 4.50 E-09 |
| "C17" | 1.32 E-09 | 7.98 E-10 |
| "C18" | 2.70 E-10 | 1.90 E-10 |
| "C19" | 2.20 E-09 | 1.40 E-09 |
| "C20" | 5.80 E-10 | 6.50 E-10 |
| "C21" | 3.70 E-08 | 1.40 E-08 |
| "C22" | 7.60 E-09 | 3.90 E-09 |
| "C23" | 5.00 E-10 | 2.90 E-10 |
| "C24" | 1.30 E-09 | 6.30 E-10 |
| "C25" | 1.80 E-09 | 8.90 E-10 |
| "C26" | 1.50 E-09 | 6.20 E-10 |
| "C27" | 4.10 E-10 | 2.70 E-10 |
| "C28" | 5.30 E-08 | 2.30 E-08 |
| "C29" | 3.40 E-09 | 3.00 E-09 |
| "C30" | 1.70 E-08 | 1.00 E-08 |
| "C31" | 3.50 E-09 | 2.60 E-09 |
| "C32" | 1.90 E-08 | 7.80 E-09 |
| "C33" | 3.30 E-09 | 1.50 E-09 |
| "C34" | 2.60 E-09 | 1.10 E-09 |
| "C35" | 4.72 E-10 | 1.92 E-10 |

| | | |
|---|---|---|
| Explanation: 3.00 E-06 means 3.00 × 10⁻⁶ | | |

The compounds shown in Table 2 are particularly preferred compounds according to the invention.

The following examples relate to medicaments:

### Example A: Injection vials

A solution of 100 g of an active ingredient of the formula I and 5 g of disodium hydrogenphosphate in 3 I of bidistilled water is adjusted to pH 6.5 using 2 N hydrochloric acid, sterile filtered, transferred into injection vials, lyophilised under sterile conditions and sealed under sterile conditions. Each injection vial contains 5 mg of active ingredient.

### Example B: Suppositories

A mixture of 20 g of an active ingredient of the formula I with 100 g of soya lecithin and 1400 g of cocoa butter is melted, poured into moulds and allowed to cool. Each suppository contains 20 mg of active ingredient.

### Example C: Solution

A solution is prepared from 1 g of an active ingredient of the formula I, 9.38 g of NaH₂PO₄ · 2 H₂O, 28.48 g of Na₂HPO₄ · 12 H₂O and 0.1 g of benzalkonium chloride in 940 ml of bidistilled water. The pH is adjusted to 6.8, and the solution is made up to 1 l and sterilised by irradiation. This solution can be used in the form of eye drops.

### Example D: Ointment

500 mg of an active ingredient of the formula I are mixed with 99.5 g of Vaseline under aseptic conditions.

### Example E: Tablets

A mixture of 1 kg of active ingredient of the formula I, 4 kg of lactose, 1.2 kg of potato starch, 0.2 kg of talc and 0.1 kg of magnesium stearate is pressed in a conventional manner to give tablets in such a way that each tablet contains 10 mg of active ingredient.

### Example F: Dragees

Tablets are pressed analogously to Example E and subsequently coated in a conventional manner with a coating of sucrose, potato starch, talc, tragacanth and dye.

### Example G: Capsules

2 kg of active ingredient of the formula I are introduced into hard gelatine capsules in a conventional manner in such a way that each capsule contains 20 mg of the active ingredient.

### Example H: Ampoules

A solution of 1 kg of active ingredient of the formula I in 60 I of bidistilled water is sterile filtered, transferred into ampoules, lyophilised under sterile conditions and sealed under sterile conditions. Each ampoule contains 10 mg of active ingredient.

## Claims

1. Compounds of the formula I 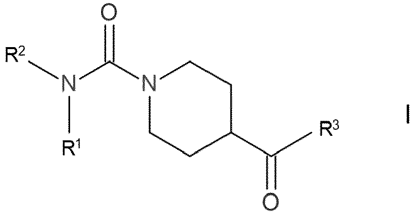 in which
R¹ denotes A,
R² denotes Ar or Het¹,
R³ denotes Het²,
Ar denotes phenyl, which is unsubstituted or mono-, di- or trisubstituted by Hal, CN, A, OR⁴, (CH₂)ₘN(R⁴)₂, SO₂N(R⁴)₂, COOR⁴ and/or CON(R⁴)₂,
Het¹ denotes pyridyl, pyrimidyl, pyrazinyl or pyridazinyl, each of which is unsubstituted or mono- or disubstituted by Hal, CN, A, NO₂, (CH₂)ₘOR⁴, (CH₂)ₘN(R⁴)₂, S(O)ₘR⁴, SO₂N(R⁴)₂, (CH₂)ₘCOOR⁴ and/or (CH₂)ₘCON(R⁴)₂,
Het² denotes benzimidazolyl, benzotriazolyl, indazolyl or indolyl, each of which is unsubstituted or mono-, di- or trisubstituted by Hal, CN, A, NO₂, (CH₂)ₘOR⁴, (CH₂)ₘN(R⁴)₂, S(O)ₘR⁴, SO₂N(R⁴)₂, (CH₂)ₘCOOR⁴ and/or (CH₂)ₘCON(R⁴)₂,
A denotes unbranched or branched alkyl with 1, 2, 3, 4, 5, 6, 7 or 8 C-Atoms, wherein one or two non-adjacent CH- and/or CH₂-groups may be replaced by N- or O-atoms and wherein 1-7 H-atoms may be replaced by F, Cl and/or OH,
R⁴ denotes H or unbranched or branched alkyl with 1, 2, 3 or 4 C-Atoms,
Hal denotes F, Cl, Br or I,
m denotes 0, 1 or 2,
and pharmaceutically acceptable solvates, salts, tautomers and stereoisomers thereof, including mixtures thereof in all ratios.

2. Compounds according to Claim 1, in which
Het¹ denotes pyridyl, which is unsubstituted or mono- or disubstituted by (CH₂)ₘCOOR⁴,
and pharmaceutically acceptable solvates, salts, tautomers and stereoisomers thereof, including mixtures thereof in all ratios.

3. Compounds according to Claim 1 or 2, in which
Ar denotes phenyl, which is unsubstituted or mono-, di- or trisubstituted by Hal, OR⁴ and/or COOR⁴,
and pharmaceutically acceptable solvates, salts, tautomers and stereoisomers thereof, including mixtures thereof in all ratios.

4. Compounds according to one or more of Claims 1-3, in which
Het² denotes benzimidazolyl, benzotriazolyl, indazolyl or indolyl, each of which is unsubstituted or mono-, di- or trisubstituted by A and/or (CH₂)ₘCOOR⁴,
and pharmaceutically acceptable solvates, salts, tautomers and stereoisomers thereof, including mixtures thereof in all ratios.

5. Compounds according to one or more of Claims 1-4, in which
A denotes A denotes unbranched or branched alkyl with 1, 2, 3, 4, 5, 6, 7 or 8 C-Atoms, wherein 1-5 H-atoms may be replaced by F,
and pharmaceutically acceptable solvates, salts, tautomers and stereoisomers thereof, including mixtures thereof in all ratios.

6. Compounds according to one or more of Claims 1-5, in which
Het¹ denotes pyridyl, which is unsubstituted or mono- or disubstituted by (CH₂)ₘCOOR⁴,
Ar denotes phenyl, which is unsubstituted or mono-, di- or trisubstituted by Hal, OR⁴ and/or COOR⁴,
Het² denotes benzimidazolyl, benzotriazolyl, indazolyl or indolyl, each of which is unsubstituted or mono-, di- or trisubstituted by A and/or (CH₂)ₘCOOR⁴,
A denotes A denotes unbranched or branched alkyl with 1, 2, 3, 4, 5, 6, 7 or 8 C-Atoms, wherein 1-5 H-atoms may be replaced by F,
and pharmaceutically acceptable solvates, salts, tautomers and stereoisomers thereof, including mixtures thereof in all ratios.

7. Compounds according to Claim 1, selected from the group
| No. | Name |
|---|---|
| "C1" | 6-{1-[(4-Fluoro-phenyl)-methyl-carbamoyl]-piperidine-4-carbonyl}-1-methyl-1H-indole-2-carboxylic acid |
| "C2" | 6-{1-[Ethyl-(4-fluoro-phenyl)-carbamoyl]-piperidine-4-carbonyl}-1-methyl-1H-indole-2-carboxylic acid |
| "C3" | 6-{1-[(4-Methoxy-phenyl)-methyl-carbamoyl]-piperidine-4-carbonyl}-1-methyl-1H-indole-2-carboxylic acid |
| "C4" | 6-{1-[Ethyl-(4-methoxy-phenyl)-carbamoyl]-piperidine-4-carbonyl}-1-methyl-1H-indole-2-carboxylic acid |
| "C5" | 5-{1-[(4-Fluoro-phenyl)-methyl-carbamoyl]-piperidine-4-carbonyl}-1-methyl-1H-indole-2-carboxylic acid |
| "C6" | 5-{1-[Ethyl-(4-fluoro-phenyl)-carbamoyl]-piperidine-4-carbonyl}-1-methyl-1H-indole-2-carboxylic acid |
| "C7" | 5-{1-[(4-Methoxy-phenyl)-methyl-carbamoyl]-piperidine-4-carbonyl}-1-methyl-1H-indole-2-carboxylic acid |
| "C8" | 5-{1-[Ethyl-(4-methoxy-phenyl)-carbamoyl]-piperidine-4-carbonyl}-1-methyl-1H-indole-2-carboxylic acid |
| "C9" | 6-{1-[(4-Fluoro-phenyl)-methyl-carbamoyl]-piperidine-4-carbonyl}-1-methyl-1H-indazole-3-carboxylic acid |
| "C 10" | 6-{1-[Ethyl-(4-fluoro-phenyl)-carbamoyl]-piperidine-4-carbonyl}-1-methyl-1H-indazole-3-carboxylic acid |
| "C11" | 6-{1-[(4-Methoxy-phenyl)-methyl-carbamoyl]-piperidine-4-carbonyl}-1-methyl-1H-indazole-3-carboxylic acid |
| "C12" | 6-{1-[Ethyl-(4-methoxy-phenyl)-carbamoyl]-piperidine-4-carbonyl}-1-methyl-1H-indazole-3-carboxylic acid |
| "C13" | 5-{1-[(4-Fluoro-phenyl)-methyl-carbamoyl]-piperidine-4-carbonyl}-1-methyl-1H-indazole-3-carboxylic acid |
| "C14" | 5-{1-[Ethyl-(4-fluoro-phenyl)-carbamoyl]-piperidine-4-carbonyl}-1-methyl-1H-indazole-3-carboxylic acid |
| "C15" | 5-{1-[(4-Methoxy-phenyl)-methyl-carbamoyl]-piperidine-4-carbonyl}-1-methyl-1H-indazole-3-carboxylic acid |
| "C16" | 5-{1-[Ethyl-(4-methoxy-phenyl)-carbamoyl]-piperidine-4-carbonyl}-1-methyl-1H-indazole-3-carboxylic acid |
| "C17" | 4-{Methyl-[4-(1-methyl-1H-indazole-6-carbonyl)-piperidine-1-carbonyl]-amino}-benzoic acid |
| "C18" | 4-{Ethyl-[4-(1-methyl-1H-indazole-6-carbonyl)-piperidine-1-carbonyl]-amino}-benzoic acid |
| "C19" | 3-{Methyl-[4-(1-methyl-1H-indazole-6-carbonyl)-piperidine-1-carbonyl]-amino}-benzoic acid |
| "C20" | 3-{Ethyl-[4-(1-methyl-1H-indazole-6-carbonyl)-piperidine-1-carbonyl]-amino}-benzoic acid |
| "C21" | 5-{Methyl-[4-(1-methyl-1H-indazole-6-carbonyl)-piperidine-1-carbonyl]-amino}-pyridine-2-carboxylic acid |
| "C22" | 4-{Methyl-[4-(1-methyl-1H-indazole-5-carbonyl)-piperidine-1-carbonyl]-amino}-benzoic acid |
| "C23" | 4-{Ethyl-[4-(1-methyl-1H-indazole-5-carbonyl)-piperidine-1-carbonyl]-amino}-benzoic acid |
| "C24" | 3-{Methyl-[4-(1-methyl-1H-indazole-5-carbonyl)-piperidine-1-carbonyl]-amino}-benzoic acid |
| "C25" | 3-{Ethyl-[4-(1-methyl-1H-indazole-5-carbonyl)-piperidine-1-carbonyl]-amino}-benzoic acid |
| "C26" | 4-{Methyl-[4-(3-methyl-3H-benzoimidazole-5-carbonyl)-piperidine-1-carbonyl]-amino}-benzoic acid |
| "C27" | 4-{Ethyl-[4-(3-methyl-3H-benzimidazole-5-carbonyl)-piperidine-1-carbonyl]-amino}-benzoic acid |
| "C28" | 3-{Methyl-[4-(3-methyl-3H-benzimidazole-5-carbonyl)-piperidine-1-carbonyl]-amino}-benzoic acid |
| "C29" | 3-{Ethyl-[4-(3-methyl-3H-benzimidazole-5-carbonyl)-piperidine-1-carbonyl]-amino}-benzoic acid |
| "C30" | 4-{Methyl-[4-(1-methyl-1H-benzimidazole-5-carbonyl)-piperidine-1-carbonyl]-amino}-benzoic acid |
| "C31" | 4-{Ethyl-[4-(1-methyl-1H-benzimidazole-5-carbonyl)-piperidine-1-carbonyl]-amino}-benzoic acid |
| "C32" | 3-{Methyl-[4-(1-methyl-1H-benzimidazole-5-carbonyl)-piperidine-1-carbonyl]-amino}-benzoic acid |
| "C33" | 3-{Ethyl-[4-(1-methyl-1H-benzimidazole-5-carbonyl)-piperidine-1-carbonyl]-amino}-benzoic acid |
| "C34" | 4-{Methyl-[4-(3-methyl-3H-benzotriazole-5-carbonyl)-piperidine-1-carbonyl]-amino}-benzoic acid |
| "C35" | 4-{Ethyl-[4-(3-methyl-3H-benzotriazole-5-carbonyl)-piperidine-1-carbonyl]-amino}-benzoic acid |
and pharmaceutically acceptable solvates, salts, tautomers and stereoisomers thereof, including mixtures thereof in all ratios.

8. Process for the preparation of pharmaceutically acceptable salts of compounds of the formula I according to Claims 1 -7 and/or of compounds of the formula I according to
Claims 1-7 wherein
R³ denotes benzimidazolyl, benzotriazolyl, indazolyl or indolyl, each of which is substituted by (CH₂)ₘCOOR⁴,
R⁴ denotes H,
and pharmaceutically acceptable salts, solvates, tautomers and stereoisomers thereof, **characterised in that** a compound of formula I, in which
R³ denotes benzimidazolyl, benzotriazolyl, indazolyl or indolyl, each of which is substituted by (CH₂)ₘCOOR⁴,
R⁴ denotes unbranched or branched alkyl with 1, 2, 3 or 4 C-Atoms,
and R¹ and R² have meanings indicated in claim 1,
is converted into another compound of formula I, in which
R³ denotes benzimidazolyl, benzotriazolyl, indazolyl or indolyl, each of which is substituted by (CH₂)ₘCOOR⁴,
R⁴ denotes H,
and R¹ and R² have meanings indicated in claim 1,
by hydrolyzing the ester;
and/or
a base or acid of the formula I is converted into one of its salts.

9. Medicaments comprising at least one compound of the formula I according to claim 1 and/or pharmaceutically acceptable salts, solvates, tautomers and stereoisomers thereof, including mixtures thereof in all ratios, and optionally an pharmaceutically acceptable carrier, excipient or vehicle.

10. Compounds of the formula I according to claim 1 and pharmaceutically acceptable salts, solvates, tautomers and stereoisomers thereof, including mixtures thereof in all ratios, for use in the treatment and/or prevention of cancer, multiple sclerosis, cardiovascular diseases, central nervous system injury and different forms of inflammation.

11. Compounds for use according to claim 10 in the treatment and/or prevention of diseases selected from the group cancer of head, neck, eye, mouth, throat, esophagus, bronchus, larynx, pharynx, chest, bone, lung, colon, rectum, stomach, prostate, urinary bladder, uterine, cervix, breast, ovaries, testicles or other reproductive organs, skin, thyroid, blood, lymph nodes, kidney, liver, pancreas, brain, central nervous system, solid tumors and blood-borne tumors.

12. Medicaments comprising at least one compound of the formula I according to claim 1 and/or pharmaceutically acceptable salts, solvates and stereoisomers thereof, including mixtures thereof in all ratios, and at least one further medicament active ingredient.

13. Set (kit) consisting of separate packs of
(a) an effective amount of a compound of the formula I according to claim 1 and/or pharmaceutically acceptable salts, solvates, and stereoisomers thereof, including mixtures thereof in all ratios,
and
(b) an effective amount of a further medicament active ingredient.

## Patentansprüche

1. Verbindungen der Formel I 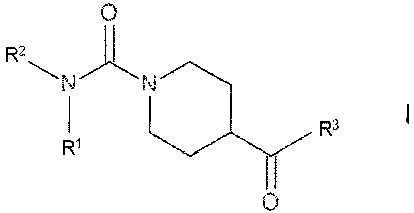 worin
R¹ A bedeutet,
R² Ar oder Het¹ bedeutet,
R³ Het² bedeutet,
Ar Phenyl bedeutet, das unsubstituiert oder ein-, zwei- oder dreifach durch Hal, CN, A, OR⁴, (CH₂)ₘN(R⁴)₂, SO₂N(R⁴)₂, COOR⁴ und/oder CON(R⁴)₂ substituiert ist,
Het¹ Pyridyl, Pyrimidyl, Pyrazinyl oder Pyridazinyl bedeutet, das jeweils unsubstituiert oder ein- oder zweifach durch Hal, CN, A, NO₂, (CH₂)ₘOR⁴, (CH₂)ₘN(R⁴)₂, S(O)ₘR⁴, SO₂N(R⁴)₂, (CH₂)ₘCOOR⁴ und/oder (CH₂)ₘCON(R⁴)₂ substituiert ist,
Het² Benzimidazolyl, Benzotriazolyl, Indazolyl oder Indolyl bedeutet, das jeweils unsubstituiert oder ein-, zwei- oder dreifach durch Hal, CN, A, NO₂, (CH₂)ₘOR⁴, (CH₂)ₘN(R⁴)₂, S(O)ₘR⁴, SO₂N(R⁴)₂, (CH₂)ₘCOOR⁴ und/oder (CH₂)ₘCON(R⁴)₂ substituiert ist,
A unverzweigtes oder verzweigtes Alkyl mit 1, 2, 3, 4, 5, 6, 7 oder 8 C-Atomen bedeutet, bei dem eine oder zwei nicht benachbarte CH-und/oder CH₂-Gruppen durch N- oder O-Atome ersetzt sein können und bei dem 1-7 H-Atome durch F, Cl und/oder OH ersetzt sein können,
R⁴ H oder unverzweigtes oder verzweigtes Alkyl mit 1, 2, 3 oder 4 C-Atomen bedeutet,
Hal F, Cl, Br oder I bedeutet,
m 0, 1 oder 2 bedeutet,
und pharmazeutisch unbedenkliche Solvate, Salze, Tautomere und Stereoisomere davon, einschließlich deren Mischungen in allen Verhältnissen.

2. Verbindungen nach Anspruch 1, worin
Het¹ Pyridyl bedeutet, das unsubstituiert oder ein- oder zweifach durch (CH₂)ₘCOOR⁴ substituiert ist,
und pharmazeutisch unbedenkliche Solvate, Salze, Tautomere und Stereoisomere davon, einschließlich deren Mischungen in allen Verhältnissen.

3. Verbindungen nach Anspruch 1 oder 2, worin
Ar Phenyl bedeutet, das unsubstituiert oder ein-, zwei- oder dreifach durch Hal, OR⁴ und/oder COOR⁴ substituiert ist,
und pharmazeutisch unbedenkliche Solvate, Salze, Tautomere und Stereoisomere davon, einschließlich deren Mischungen in allen Verhältnissen.

4. Verbindungen nach einem oder mehreren der Ansprüche 1-3, worin
Het² Benzimidazolyl, Benzotriazolyl, Indazolyl oder Indolyl bedeutet, das jeweils unsubstituiert oder ein-, zwei- oder dreifach durch A und/oder (CH₂)ₘCOOR⁴ substituiert ist,
und pharmazeutisch unbedenkliche Solvate, Salze, Tautomere und Stereoisomere davon, einschließlich deren Mischungen in allen Verhältnissen.

5. Verbindungen nach einem oder mehreren der Ansprüche 1-4, worin
A unverzweigtes oder verzweigtes Alkyl mit 1, 2, 3, 4, 5, 6, 7 oder 8 C-Atomen bedeutet, bei dem 1-5 H-Atome durch F ersetzt sein können,
und pharmazeutisch unbedenkliche Solvate, Salze, Tautomere und Stereoisomere davon, einschließlich deren Mischungen in allen Verhältnissen.

6. Verbindungen nach einem oder mehreren der Ansprüche 1-5, worin
Het¹ Pyridyl bedeutet, das unsubstituiert oder ein- oder zweifach durch (CH₂)ₘCOOR⁴ substituiert ist,
Ar Phenyl bedeutet, das unsubstituiert oder ein-, zwei- oder dreifach durch Hal, OR⁴ und/oder COOR⁴ substituiert ist,
Het² Benzimidazolyl, Benzotriazolyl, Indazolyl oder Indolyl bedeutet, das jeweils unsubstituiert oder ein-, zwei- oder dreifach durch A und/oder (CH₂)ₘCOOR⁴ substituiert ist,
A unverzweigtes oder verzweigtes Alkyl mit 1, 2, 3, 4, 5, 6, 7 oder 8 C-Atomen bedeutet, bei dem 1-5 H-Atome durch F ersetzt sein können,
und pharmazeutisch unbedenkliche Solvate, Salze, Tautomere und Stereoisomere davon, einschließlich deren Mischungen in allen Verhältnissen.

7. Verbindungen nach Anspruch 1, ausgewählt aus der Gruppe
| Nr. | Name |
|---|---|
| "C1" | 6-{1-[(4-Fluor-phenyl)-methyl-carbamoyl]-piperidin-4-carbonyl}-1-methyl-1H-indol-2-carbonsäure |
| "C2" | 6-{1-[Ethyl-(4-fluor-phenyl)-carbamoyl]-piperidin-4-carbonyl}-1-methyl-1H-indol-2-carbonsäure |
| "C3" | 6-{1-[(4-Methoxy-phenyl)-methyl-carbamoyl]-piperidin-4-carbonyl}-1-methyl-1H-indol-2-carbonsäure |
| "C4" | 6-{1-[Ethyl-(4-methoxy-phenyl)-carbamoyl]-piperidin-4-carbonyl}-1-methyl-1H-indol-2-carbonsäure |
| "C5" | 5-{1-[(4-Fluor-phenyl)-methyl-carbamoyl]-piperidin-4-carbonyl}-1-methyl-1H-indol-2-carbonsäure |
| "C6" | 5-{1-[Ethyl-(4-fluor-phenyl)-carbamoyl]-piperidin-4-carbonyl}-1-methyl-1H-indol-2-carbonsäure |
| "C7" | 5-{1-[(4-Methoxy-phenyl)-methyl-carbamoyl]-piperidin-4-carbonyl}-1-methyl-1H-indol-2-carbonsäure |
| "C8" | 5-{1-[Ethyl-(4-methoxy-phenyl)-carbamoyl]-piperidin-4-carbonyl}-1-methyl-1H-indol-2-carbonsäure |
| "C9" | 6-{1-[(4-Fluor-phenyl)-methyl-carbamoyl]-piperidin-4-carbonyl}-1-methyl-1H-indazol-3-carbonsäure |
| "C10" | 6-{1-[Ethyl-(4-fluor-phenyl)-carbamoyl]-piperidin-4-carbonyl}-1-methyl-1H-indazol-3-carbonsäure |
| "C11" | 6-{1-[(4-Methoxy-phenyl)-methyl-carbamoyl]-piperidin-4-carbonyl}-1-methyl-1H-indazol-3-carbonsäure |
| "C12" | 6-{1-[Ethyl-(4-methoxy-phenyl)-carbamoyl]-piperidin-4-carbonyl}-1-methyl-1H-indazol-3-carbonsäure |
| "C13" | 5-{1-[(4-Fluor-phenyl)-methyl-carbamoyl]-piperidin-4-carbonyl}-1-methyl-1H-indazol-3-carbonsäure |
| "C14" | 5-{1-[Ethyl-(4-fluor-phenyl)-carbamoyl]-piperidin-4-carbonyl}-1-methyl-1H-indazol-3-carbonsäure |
| "C15" | 5-{1-[(4-Methoxy-phenyl)-methyl-carbamoyl]-piperidin-4-carbonyl}-1-methyl-1H-indazol-3-carbonsäure |
| "C16" | 5-{1-[Ethyl-(4-methoxy-phenyl)-carbamoyl]-piperidin-4-carbonyl}-1-methyl-1H-indazol-3-carbonsäure |
| "C17" | 4-{Methyl-[4-(1-methyl-1H-indazol-6-carbonyl)-piperidin-1-carbonyl]-amino}-benzoesäure |
| "C18" | 4-{Ethyl-[4-(1-methyl-1H-indazol-6-carbonyl)-piperidin-1-carbonyl]-amino}-benzoesäure |
| "C19" | 3-{Methyl-[4-(1-methyl-1H-indazol-6-carbonyl)-piperidin-1-carbonyl]-amino}-benzoesäure |
| "C20" | 3-{Ethyl-[4-(1-methyl-1H-indazol-6-carbonyl)-piperidin-1-carbonyl]-amino}-benzoesäure |
| "C21" | 5-{Methyl-[4-(1-methyl-1H-indazol-6-carbonyl)-piperidin-1-carbonyl]-amino}-pyridin-2-carbonsäure |
| "C22" | 4-{Methyl-[4-(1-methyl-1H-indazol-5-carbonyl)-piperidin-1-carbonyl]-amino}-benzoesäure |
| "C23" | 4-{Ethyl-[4-(1-methyl-1H-indazol-5-carbonyl)-piperidin-1-carbonyl]-amino}-benzoesäure |
| "C24" | 3-{Methyl-[4-(1-methyl-1H-indazol-5-carbonyl)-piperidin-1-carbonyl]-amino}-benzoesäure |
| "C25" | 3-{Ethyl-[4-(1-methyl-1H-indazol-5-carbonyl)-piperidin-1-carbonyl]-amino}-benzoesäure |
| "C26" | 4-{Methyl-[4-(3-methyl-3H-benzoimidazol-5-carbonyl)-piperidin-1-carbonyl]-amino}-benzoesäure |
| "C27" | 4-{Ethyl-[4-(3-methyl-3H-benzimidazol-5-carbonyl)-piperidin-1-carbonyl]-amino}-benzoesäure |
| "C28" | 3-{Methyl-[4-(3-methyl-3H-benzimidazol-5-carbonyl)-piperidin-1-carbonyl]-amino}-benzoesäure |
| "C29" | 3-{Ethyl-[4-(3-methyl-3H-benzimidazol-5-carbonyl)-piperidin-1-carbonyl]-amino}-benzoesäure |
| "C30" | 4-{Methyl-[4-(1-methyl-1H-benzimidazol-5-carbonyl)-piperidin-1-carbonyl]-amino}-benzoesäure |
| "C31" | 4-{Ethyl-[4-(1-methyl-1H-benzimidazol-5-carbonyl)-piperidin-1-carbonyl]-amino}-benzoesäure |
| "C32" | 3-{Methyl-[4-(1-methyl-1H-benzimidazol-5-carbonyl)-piperidin-1-carbonyl]-amino}-benzoesäure |
| "C33" | 3-{Ethyl-[4-(1-methyl-1H-benzimidazol-5-carbonyl)-piperidin-1-carbonyl]-amino}-benzoesäure |
| "C34" | 4-{Methyl-[4-(3-methyl-3H-benzotriazol-5-carbonyl)-piperidin-1-carbonyl]-amino}-benzoesäure |
| "C35" | 4-{Ethyl-[4-(3-methyl-3H-benzotriazol-5-carbonyl)-piperidin-1-carbonyl]-amino}-benzoesäure |
und pharmazeutisch unbedenkliche Solvate, Salze, Tautomere und Stereoisomere davon, einschließlich deren Mischungen in allen Verhältnissen.

8. Verfahren zur Herstellung von pharmazeutisch unbedenklichen Salzen von Verbindungen der Formel I nach den Ansprüchen 1-7 und/oder von Verbindungen der Formel I nach den Ansprüchen 1-7, bei denen
R³ Benzimidazolyl, Benzotriazolyl, Indazolyl oder Indolyl bedeutet, das jeweils durch (CH₂)ₘCOOR⁴ substituiert ist,
R⁴ H bedeutet,
und pharmazeutisch unbedenklichen Salzen, Solvaten, Tautomeren und Stereoisomeren davon, **dadurch gekennzeichnet, dass** man eine Verbindung der Formel I, worin
R³ Benzimidazolyl, Benzotriazolyl, Indazolyl oder Indolyl bedeutet, das jeweils durch (CH₂)ₘCOOR⁴ substituiert ist,
R⁴ unverzweigtes oder verzweigtes Alkyl mit 1, 2, 3 oder 4 C-Atomen bedeutet,
und R¹ und R² in Anspruch 1 angegebene Bedeutungen besitzen,
durch Hydrolysieren des Esters in eine andere Verbindung der Formel I umwandelt, worin
R³ Benzimidazolyl, Benzotriazolyl, Indazolyl oder Indolyl bedeutet, das jeweils durch (CH₂)ₘCOOR⁴ substituiert ist,
R⁴ H bedeutet,
und R¹ und R² in Anspruch 1 angegebene Bedeutungen besitzen;
und/oder
eine Base oder Säure der Formel I in eines ihrer Salze umwandelt.

9. Arzneimittel enthaltend mindestens eine Verbindung der Formel I nach Anspruch 1 und/oder pharmazeutisch unbedenkliche Salze, Solvate, Tautomere und Stereoisomere davon, einschließlich deren Mischungen in allen Verhältnissen, sowie gegebenenfalls ein(en) pharmazeutisch unbedenklichen/s Trägerstoff, Exzipienten oder Vehikel.

10. Verbindungen der Formel I nach Anspruch 1 und pharmazeutisch unbedenkliche Salze, Solvate, Tautomere und Stereoisomere davon, einschließlich deren Mischungen in allen Verhältnissen, zur Verwendung bei der Behandlung und/oder Prävention von Krebs, multipler Sklerose, Herz-Kreislauf-Erkrankungen, Verletzungen des Zentralnervensystems und verschiedenen Formen der Entzündung.

11. Verbindungen zur Verwendung nach Anspruch 10 bei der Behandlung und/oder Prävention von Erkrankungen, die ausgewählt sind aus der Gruppe Krebs von Kopf, Hals, Auge, Mund, Kehle, Speiseröhre, Bronchie, Kehlkopf, Rachenhöhle, Brustkorb, Knochen, Lunge, Kolon, Rektum, Magen, Prostata, Harnblase, Gebärmutter, Muttermund, Brust, Eierstöcken, Hoden oder anderen Fortpflanzungsorganen, Haut, Schilddrüse, Blut, Lymphknoten, Niere, Leber, Bauchspeicheldrüse, Hirn, Zentralnervensystem, feste Tumoren und hämatogene Tumoren.

12. Arzneimittel enthaltend mindestens eine Verbindung der Formel I nach Anspruch 1 und/oder pharmazeutisch unbedenkliche Salze, Solvate und Stereoisomere davon, einschließlich deren Mischungen in allen Verhältnissen, und mindestens einen weiteren Arzneimittelwirkstoff.

13. Set (Kit) bestehend aus getrennten Packungen von
(a) einer wirksamen Menge einer Verbindung der Formel I nach Anspruch 1 und/oder pharmazeutisch unbedenklicher Salze, Solvate und Stereoisomere davon, einschließlich deren Mischungen in allen Verhältnissen,
und
(b) einer wirksamen Menge eines weiteren Arzneimittelwirkstoffs.

## Revendications

1. Composés de formule I 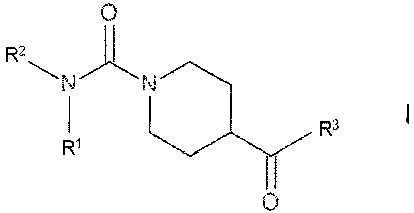 dans lesquels
R¹ désigne A,
R² désigne Ar ou Het¹,
R³ désigne Het²,
Ar désigne phényle, qui est non substitué ou mono-, di- ou trisubstitué par Hal, CN, A, OR⁴, (CH₂)ₘN(R⁴)₂, SO₂N(R⁴)₂, COOR⁴ et/ou CON(R⁴)₂,
Het¹ désigne pyridyle, pyrimidyle, pyrazinyle ou pyridazinyle, chacun d'entre eux étant non substitué ou mono- ou disubstitué par Hal, CN, A, NO₂, (CH₂)ₘOR⁴, (CH₂)ₘN(R⁴)₂, S(O)ₘR⁴, SO₂N(R⁴)₂, (CH₂)ₘCOOR⁴ et/ou (CH₂)ₘCON(R⁴)₂,
Het² désigne benzimidazolyle, benzotriazolyle, indazolyle ou indolyle, chacun d'entre eux étant non substitué ou mono-, di- ou trisubstitué par Hal, CN, A, NO₂, (CH₂)ₘOR⁴, (CH₂)ₘN(R⁴)₂, S(O)ₘR⁴, SO₂N(R⁴)₂, (CH₂)ₘCOOR⁴ et/ou (CH₂)ₘCON(R⁴)₂,
A désigne alkyle non ramifié ou ramifié ayant 1, 2, 3, 4, 5, 6, 7 ou 8 atomes de C, où un ou deux groupements CH et/ou CH₂ non adjacents peuvent être remplacés par des atomes de N ou O et où 1-7 atomes de H peuvent être remplacés par F, Cl et/ou OH,
R⁴ désigne H ou alkyle non ramifié ou ramifié ayant 1, 2, 3 ou 4 atomes de C,
Hal désigne F, Cl, Br ou I,
m désigne 0, 1 ou 2,
ainsi que les solvates, sels, tautomères et stéréoisomères pharmaceutiquement acceptables de ceux-ci, y compris des mélanges de ceux-ci en toutes proportions.

2. Composés selon la revendication 1, dans lesquels
Het¹ désigne pyridyle, qui est non substitué ou mono- ou disubstitué par (CH₂)ₘCOOR⁴,
ainsi que les solvates, sels, tautomères et stéréoisomères pharmaceutiquement acceptables de ceux-ci, y compris des mélanges de ceux-ci en toutes proportions.

3. Composés selon la revendication 1 ou 2, dans lesquels
Ar désigne phényle, qui est non substitué ou mono-, di- ou trisubstitué par Hal, OR⁴ et/ou COOR⁴,
ainsi que les solvates, sels, tautomères et stéréoisomères pharmaceutiquement acceptables de ceux-ci, y compris des mélanges de ceux-ci en toutes proportions.

4. Composés selon l'une ou plusieurs parmi les revendications 1-3, dans lesquels
Het² désigne benzimidazolyle, benzotriazolyle, indazolyle ou indolyle, chacun d'entre eux étant non substitué ou mono-, di- ou trisubstitué par A et/ou (CH₂)ₘCOOR⁴,
ainsi que les solvates, sels, tautomères et stéréoisomères pharmaceutiquement acceptables de ceux-ci, y compris des mélanges de ceux-ci en toutes proportions.

5. Composés selon l'une ou plusieurs parmi les revendications 1-4, dans lesquels
A désigne alkyle non ramifié ou ramifié ayant 1, 2, 3, 4, 5, 6, 7 ou 8 atomes de C, où 1-5 atomes de H peuvent être remplacés par F,
ainsi que les solvates, sels, tautomères et stéréoisomères pharmaceutiquement acceptables de ceux-ci, y compris des mélanges de ceux-ci en toutes proportions.

6. Composés selon l'une ou plusieurs parmi les revendications 1-5, dans lesquels
Het¹ désigne pyridyle, qui est non substitué ou mono- ou disubstitué par (CH₂)ₘCOOR⁴,
Ar désigne phényle, qui est non substitué ou mono-, di- ou trisubstitué par Hal, OR⁴ et/ou COOR⁴,
Het² désigne benzimidazolyle, benzotriazolyle, indazolyle ou indolyle, chacun d'entre eux étant non substitué ou mono-, di- ou trisubstitué par A et/ou (CH₂)ₘCOOR⁴,
A désigne alkyle non ramifié ou ramifié ayant 1, 2, 3, 4, 5, 6, 7 ou 8 atomes de C, où 1-5 atomes de H peuvent être remplacés par F,
ainsi que les solvates, sels, tautomères et stéréoisomères pharmaceutiquement acceptables de ceux-ci, y compris des mélanges de ceux-ci en toutes proportions.

7. Composés selon la revendication 1, choisis dans le groupe constitué par
| N° | Nom |
|---|---|
| « C1 » | Acide 6-{1-[(4-fluorophényl)méthylcarbamoyl]pipéridine-4-carbonyl}-1-méthyl-1H-indole-2-carboxylique |
| « C2 » | Acide 6-{1-[éthyl-(4-fluorophényl)carbamoyl]pipéridine-4-carbonyl}-1-méthyl-1H-indole-2-carboxylique |
| « C3 » | Acide 6-{1-[(4-méthoxyphényl)méthylcarbamoyl]pipéridine-4-carbonyl}-1-méthyl-1H-indole-2-carboxylique |
| « C4 » | Acide 6-{1-[éthyl-(4-méthoxyphényl)carbamoyl]pipéridine-4-carbonyl}-1-méthyl-1H-indole-2-carboxylique |
| « C5 » | Acide 5-{1-[(4-fluorophényl)méthylcarbamoyl]pipéridine-4-carbonyl}-1-méthyl-1H-indole-2-carboxylique |
| « C6 » | Acide 5-{1-[éthyl-(4-fluorophényl)carbamoyl]pipéridine-4-carbonyl}-1-méthyl-1H-indole-2-carboxylique |
| « C7 » | Acide 5-{1-[(4-méthoxyphényl)méthylcarbamoyl]pipéridine-4-carbonyl}-1-méthyl-1H-indole-2-carboxylique |
| « C8 » | Acide 5-{1-[éthyl-(4-méthoxyphényl)carbamoyl]pipéridine-4-carbonyl}-1-méthyl-1H-indole-2-carboxylique |
| « C9 » | Acide 6-{1-[(4-fluorophényl)méthylcarbamoyl]pipéridine-4-carbonyl}-1-méthyl-1H-indazole-3-carboxylique |
| « C10 » | Acide 6-{1-[éthyl-(4-fluorophényl)carbamoyl]pipéridine-4-carbonyl}-1-méthyl-1H-indazole-3-carboxylique |
| « C11 » | Acide 6-{1-[(4-méthoxyphényl)méthylcarbamoyl]pipéridine-4-carbonyl}-1-méthyl-1H-indazole-3-carboxylique |
| « C12 » | Acide 6-{1-[éthyl-(4-méthoxyphényl)carbamoyl]pipéridine-4-carbonyl}-1-méthyl-1H-indazole-3-carboxylique |
| « C13 » | Acide 5-{1-[(4-fluorophényl)méthylcarbamoyl]pipéridine-4-carbonyl}-1-méthyl-1H-indazole-3-carboxylique |
| « C14 » | Acide 5-{1-[éthyl-(4-fluorophényl)carbamoyl]pipéridine-4-carbonyl}-1-méthyl-1H-indazole-3-carboxylique |
| « C15 » | Acide 5-{1-[(4-méthoxyphényl)méthylcarbamoyl]pipéridine-4-carbonyl}-1-méthyl-1H-indazole-3-carboxylique |
| « C16 » | Acide 5-{1-[éthyl-(4-méthoxyphényl)carbamoyl]pipéridine-4-carbonyl}-1-méthyl-1H-indazole-3-carboxylique |
| « C17 » | Acide 4-{méthyl-[4-(1-méthyl-1H-indazole-6-carbonyl)-pipéridine-1-carbonyl]amino}benzoïque |
| « C18 » | Acide 4-{éthyl-[4-(1-méthyl-1H-indazole-6-carbonyl)-pipéridine-1-carbonyl]amino}benzoïque |
| "C19" | Acide 3-{méthyl-[4-(1-méthyl-1H-indazole-6-carbonyl)-pipéridine-1-carbonyl]amino}benzoïque |
| « C20 » | Acide 3-{éthyl-[4-(1-méthyl-1H-indazole-6-carbonyl)-pipéridine-1-carbonyl]amino}benzoïque |
| « C21 » | Acide 5-{méthyl-[4-(1-méthyl-1H-indazole-6-carbonyl)-pipéridine-1-carbonyl]amino}-pyridine-2-carboxylique |
| « C22 » | Acide 4-{méthyl-[4-(1-méthyl-1H-indazole-5-carbonyl)-pipéridine-1-carbonyl]amino}benzoïque |
| « C23 » | Acide 4-{éthyl-[4-(1-méthyl-1H-indazole-5-carbonyl)-pipéridine-1-carbonyl]amino}benzoïque |
| « C24 » | Acide 3-{méthyl-[4-(1-méthyl-1H-indazole-5-carbonyl)-pipéridine-1-carbonyl]amino}benzoïque |
| « C25 » | Acide 3-{éthyl-[4-(1-méthyl-1H-indazole-5-carbonyl)-pipéridine-1-carbonyl]amino}benzoïque |
| « C26 » | Acide 4-{méthyl-[4-(3-méthyl-3H-benzoimidazole-5-carbonyl)pipéridine-1-carbonyl]amino}benzoïque |
| « C27 » | Acide 4-{éthyl-[4-(3-méthyl-3H-benzimidazole-5-carbonyl)-pipéridine-1-carbonyl]amino}benzoïque |
| « C28 » | Acide 3-{méthyl-[4-(3-méthyl-3H-benzimidazole-5-carbonyl)-pipéridine-1-carbonyl]amino}benzoïque |
| « C29 » | Acide 3-{éthyl-[4-(3-méthyl-3H-benzimidazole-5-carbonyl)-pipéridine-1-carbonyl]amino}benzoïque |
| « C30 » | Acide 4-{méthyl-[4-(1-méthyl-1H-benzimidazole-5-carbonyl)-pipéridine-1-carbonyl]amino}benzoïque |
| « C31 » | Acide 4-{éthyl-[4-(1-méthyl-1H-benzimidazole-5-carbonyl)-pipéridine-1-carbonyl]amino}benzoïque |
| « C32 » | Acide 3-{méthyl-[4-(1-méthyl-1H-benzimidazole-5-carbonyl)-pipéridine-1-carbonyl]amino}benzoïque |
| « C33 » | Acide 3-{éthyl-[4-(1-méthyl-1H-benzimidazole-5-carbonyl)-pipéridine-1-carbonyl]amino}benzoïque |
| « C34 » | Acide 4-{méthyl-[4-(3-méthyl-3H-benzotriazole-5-carbonyl)-pipéridine-1-carbonyl]amino}benzoïque |
| « C35 » | Acide 4-{éthyl-[4-(3-méthyl-3H-benzotriazole-5-carbonyl)-pipéridine-1-carbonyl]amino}benzoïque |
ainsi que les solvates, sels, tautomères et stéréoisomères pharmaceutiquement acceptables de ceux-ci, y compris des mélanges de ceux-ci en toutes proportions.

8. Procédé de préparation de sels pharmaceutiquement acceptables de composés de formule I selon les revendications 1-7 et/ou de composés de formule I selon les revendications 1-7, dans lesquels
R³ désigne benzimidazolyle, benzotriazolyle, indazolyle ou indolyle, chacun d'entre eux étant substitué par (CH₂)ₘCOOR⁴,
R⁴ désigne H,
ainsi que les sels, solvates, tautomères et stéréoisomères pharmaceutiquement acceptables de ceux-ci, **caractérisé en ce que**
un composé de formule I, dans lequel
R³ désigne benzimidazolyle, benzotriazolyle, indazolyle ou indolyle, chacun d'entre eux étant substitué par (CH₂)ₘCOOR⁴,
R⁴ désigne alkyle non ramifié ou ramifié ayant 1, 2, 3 ou 4 atomes de C,
et R¹ et R² revêtent les significations indiquées selon la revendication 1,
est converti en un autre composé de formule I, dans lequel
R³ désigne benzimidazolyle, benzotriazolyle, indazolyle ou indolyle, chacun d'entre eux étant substitué par (CH₂)ₘCOOR⁴,
R⁴ désigne H,
et R¹ et R² revêtent les significations indiquées selon la revendication 1, par l'hydrolyse de l'ester ;
et/ou
une base ou un acide de formule I est converti(e) en l'un de ses sels.

9. Médicaments comprenant au moins un composé de formule I selon la revendication 1 et/ou des sels, solvates, tautomères et stéréoisomères pharmaceutiquement acceptables de celui-ci, y compris des mélanges de ceux-ci en toutes proportions, et éventuellement un support, un excipient ou un véhicule pharmaceutiquement acceptable.

10. Composés de formule I selon la revendication 1 ainsi que les sels, solvates, tautomères et stéréoisomères pharmaceutiquement acceptables de ceux-ci, y compris des mélanges de ceux-ci en toutes proportions, pour une utilisation dans le traitement et/ou la prévention du cancer, de la sclérose en plaques, des maladies cardiovasculaires, des lésions du système nerveux central et de différentes formes d'inflammations.

11. Composés pour une utilisation selon la revendication 10, dans le traitement et/ou la prévention de maladies choisies dans le groupe constitué par le cancer de la tête, du cou, de l'œil, de la bouche, de la gorge, de l'oesophage, des bronches, du larynx, du pharynx, de la poitrine, des os, du poumon, du côlon, du rectum, de l'estomac, de la prostate, de la vessie urinaire, de l'utérus, du col de l'utérus, du sein, des ovaires, des testicules ou autres organes reproducteurs, de la peau, de la thyroïde, du sang, des noeuds lymphoïdes, du rein, du foie, du pancréas, du cerveau, du système nerveux central, les tumeurs solides et les tumeurs du sang.

12. Médicaments comprenant au moins un composé de formule I selon la revendication 1 et/ou des sels, solvates et stéréoisomères pharmaceutiquement acceptables de celui-ci, y compris des mélanges de ceux-ci en toutes proportions, et au moins un autre ingrédient actif médicamenteux.

13. Ensemble (kit) constitué de conditionnements séparés
(a) d'une quantité efficace d'un composé de formule I selon la revendication 1 et/ou de sels, solvates, et stéréoisomères pharmaceutiquement acceptables de celui-ci, y compris des mélanges de ceux-ci en toutes proportions,
et
(b) d'une quantité efficace d'un autre ingrédient actif médicamenteux.
